# EUROPÄISCHE PATENTANMELDUNG

(11) **EP 2 712 934 A2**
(43) Veröffentlichungstag der Anmeldung: **02.04.2014**
(21) Anmeldenummer: 13190162.1
(22) Anmeldetag: 06.07.2009
(51) Int. Cl.: C12Q 1/68, G01N 33/53

(54) **Markersequenzen für Prostataentzündungserkrankungen, Prostatakarzinom und deren Verwendung**

(30) Priorität: 04.07.2008 DE 102008031699
(62) Teilanmeldung aus: 09772574.1
(71) Anmelder: Protagen AG, 44227 Dortmund (DE)
(72) Erfinder: Lüking, Angelika, 44892 Bochum (DE); Kowald, Axel, 44892 Bochum (DE); Beator, Jens, 37547 Kreiensen (DE); Klocker, Helmut, 6401 Inzing (AT); Bartsch, Georg, 6072 Lans (AT); Meyer, Helmut E., 45661 Recklinghausen (DE)
(74) Vertreter: Simandi, Claus

(57) **Zusammenfassung**

Die vorliegende Erfindung betrifft neue Markersequenzen für Prostataentzündungserkrankungen, Prostatakarzinom und deren diagnostische Verwendung samt einem Verfahren zum Screenen von potentiellen Wirkstoffen für solche Prostata - Erkrankungen mittels dieser Markersequenzen. Ferner betrifft die Erfindung eine diagnostische Vorrichtung enthaltend solche Markersequenzen für Prostataentzündungserkrankungen, Prostatakarzinom, insbesondere ein Proteinbiochip und dessen Verwendung.

## Beschreibung

Die vorliegende Erfindung betrifft neue Markersequenzen für Prostataentzündungserkrankungen, Prostatakarzinom und deren diagnostische Verwendung samt einem Verfahren zum Screenen von potentiellen Wirkstoffen für solche Prostata - Erkrankungen mittels dieser Markersequenzen. Ferner betrifft die Erfindung eine diagnostische Vorrichtung enthaltend solche Markersequenzen für Prostataentzündungserkrankungen, Prostatakarzinom, insbesondere ein Proteinbiochip und dessen Verwendung.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Es besteht jedoch ein hohes Bedürfnis indikationsspezifische diagnostische Vorrichtungen, wie einen Proteinbiochip, bereitzustellen.

Zu den Laborparametern gehören die saure Phosphatase (SP) und das prostataspezifische Antigen (PSA) zur Diagnose des Prostatakarzinom. Vor allem das PSA hat derzeit einen hohen Stellenwert in der Diagnostik. Es ist spezifisch für die Prostata, allerdings nicht für ein Tumorleiden, sondern kann auch bei Entzündungen, benigner Prostatahyperplasie, einem Harnverhalt oder ohne ersichtlichen Grund erhöht sein. Ein Wert über 4 ng/ml gilt bereits als abklärungsbedürftig.

Die Aufgabe der vorliegenden Erfindung ist die Bereitstellung von verbesserten Markersequenzen und deren diagnostische Verwendung zur Behandlung von Prostataentzündungserkrankungen bis hin zum Prostatakarzinom.

Die Bereitstellung von spezifischen Markersequenzen erlaubt eine sichere Diagnose und Stratifizierung von Patienten mit Prostataentzündungserkrankungen bis hin zum Prostatakarzinom, insbesondere mittels eines Proteinbiochips.

Daher betrifft die Erfindung die Verwendung von Markersequenzen zur Diagnose von Prostataentzündungserkrankungen bis hin zum Prostatakarzinom, besonders bevorzugt Prostatakarzinom, wobei mindestens eine Markersequenz einer cDNA ausgewählt aus der Gruppe SEQ 1 - 174 oder jeweils ein dafür kodierendes Protein oder jeweils einer Teilsequenz oder Fragment davon (nachstehend: erfindungsgemäße Markersequenzen) an oder zu einem zu untersuchenden Patienten bestimmt wird.

Die erfindungsgemäßen Markersequenzen konnten mittels differentiellem Screenen von Proben und zwar gesunder Probanden mit Patientenproben mit Prostataentzündungserkrankungen, Prostatakarzinom identifiziert werden.

Hierbei konnte erstmals mittels Proteinbiochips (siehe Beispiele) diese erfindungsgemäßen Markersequenzen identifiziert werden.

Der Begriff "Prostataentzündungserkrankungen bis hin zum Prostatakarzinom" umfasst eine Gruppe von Erkrankungen von Prostatis bis hin zu den chronischen Formen sämtlicher Prostataentzündungen und deren Etablierung als Prostatakrebs oder Prostatakarzimon (Definition z.B. nach Pschyrembel, de Gruyter, 261. Auflage (2007), Berlin).

In einer weiteren Ausführungsform werden daher mindestens 2 bis 5 oder 10, vorzugsweise 30 bis 50 Markersequenzen oder 50 bis 100 oder mehr Markersequenzen an oder zu einem zu untersuchenden Patienten bestimmt.

In einer weiteren Ausführungsform der Erfindung können die erfindungsgemäßen Markersequenzen ebenfalls mit bekannten Biomarkern für diese Indikation kombiniert, ergänzt oder erweitert werden.

In einer bevorzugten Ausführungsform erfolgt die Bestimmung der Markersequenzen außerhalb des menschlichen Körpers und die Bestimmung erfolgt in einer ex vivo / in vitro Diagnose.

In einer weiteren Ausführungsform der Erfindung betrifft die Erfindung die Verwendung von Markersequenzen als Diagnostika, wobei mindestens eine Markersequenz einer cDNA ausgewählt aus der Gruppe SEQ 1 - 174 oder jeweils ein dafür kodierendes Protein oder jeweils einer Teilsequenz oder Fragment davon ist.

Ferner betrifft die Erfindung ein Verfahren zur Diagnose von Prostataentzündungserkrankungen bis hin zum Prostatakarzinom, wobei a.) mindestens eine Markersequenz einer cDNA ausgewählt aus der Gruppe SEQ 1 - 174 oder jeweils ein dafür kodierendes Protein oder jeweils einer Teilsequenz oder Fragment davon auf einem festen Träger aufgebracht wird und b.) mit Körperflüssigkeit oder Gewebeauszug eines Patienten in Kontakt gebracht wird und c.) der Nachweis einer Wechselwirkung der Körperflüssigkeit oder Gewebeauszug mit den Markersequenzen aus a.) erfolgt.

Daher betrifft die Erfindung ebenfalls Diagnostika zur Diagnose von Prostataentzündungserkrankungen bis hin zum Prostatakarzinom jeweils ausgewählt aus der Gruppe SEQ 1 - 174 oder jeweils ein dafür kodierendes Protein oder jeweils einer Teilsequenz oder Fragment davon.

In einer besonders bevorzugten Ausführungsform sind die Markersequenzen SEQ 136, 40, 127, 83, 16, 82, 88, 152, 130, 138, 2, 12, 113, 20, 173, 33, 172, 52, 43, 91, 1, 32, 86, 27, 105 in dieser Reihenfolge bevorzugt.

Der Nachweis einer solchen Wechselwirkung kann beispielsweise durch eine Sonde, insbesondere durch einen Antikörper erfolgen.

Daher betrifft die Erfindung ebenfalls die Aufgabe eine diagnostische Vorrichtung oder einen Assay, insbesondere einen Proteinbiochip, bereitzustellen, der für die Prostataentzündungserkrankungen bis hin zum Prostatakarzinom eine Diagnose oder Untersuchung erlaubt.

Ferner betrifft die Erfindung ein Verfahren zum Stratifizieren, insbesondere zur Risikostratifizierung und / oder Therapiesteuerung eines Patienten mit Prostataentzündungserkrankungen bis hin zum Prostatakarzinom, wobei mindestens eine Markersequenz einer cDNA ausgewählt aus der Gruppe SEQ 1 - 174 oder jeweils ein dafür kodierendes Protein an einem zu untersuchenden Patienten bestimmt wird.

Ferner umfasst ist die Stratifizierung der Patienten mit Prostataentzündungserkrankungen bis hin zum Prostatakarzinom in neue oder etablierte Subgruppen der Prostataentzündungserkrankungen bis hin zum Prostatakarzinom, sowie die sinnvolle Auswahl von Patientengruppen für die klinische Entwicklung von neuen Therapeutika. Der Begriff Therapiesteuerung umfasst ebenfalls die Einteilung von Patienten in Responder und Nicht-Responder bezüglich einer Therapie oder dessen Therapieverlauf.

"Diagnose" im Sinne dieser Erfindung bedeutet die positive Feststellung der Prostataentzündungserkrankungen bis hin zum Prostatakarzinom mittels der erfindungsgemäßen Markersequenzen sowie die Zuordnung der Patienten zu den Prostataentzündungserkrankungen bis hin zum Prostatakarzinom. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose ebenfalls die Differentialdiagnose von Prostataentzündungserkrankungen, Prostatakarzinom mittels der erfindungsgemäßen Markersequenzen sowie die Prognose der Prostataentzündungserkrankungen, Prostatakarzinom.

"Stratifizieren (auch: Stratifikation) oder Therapiesteuerung" im Sinne dieser Erfindung bedeutet, dass das erfindungsgemäße Verfahren Entscheidungen zur Behandlung und Therapie des Patienten erlaubt, sei es Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Prostataentzündungserkrankungen bis hin zum Prostatakarzinom untersucht wird.

Der Begriff "Markersequenzen" im Sinne dieser Erfindung bedeutet, dass die cDNA oder das jeweils daraus erhältliche Polypeptid oder Protein signifikant für Prostataentzündungserkrankungen, Prostatakarzinom sind. Beispielsweise können die cDNA oder das jeweils daraus erhältliche Polypeptid oder Protein eine Wechselwirkung mit Substanzen aus der Körperflüssigkeit oder Gewebeauszug eines Patienten mit Prostataentzündungserkrankungen bis hin zum Prostatakarzinom aufweisen (z.B. Antigen (Epitop) / Antikörper (Paratop) Wechselwirkung). Im Sinne der Erfindung bedeutet "wobei mindestens eine Markersequenz einer cDNA ausgewählt aus der Gruppe SEQ 1 - 174 oder jeweils ein dafür kodierendes Protein oder jeweils einer Teilsequenz oder Fragment davon an einem zu untersuchenden Patienten bestimmt wird", dass eine Wechselwirkung zwischen der Körperflüssigkeit oder Gewebeauszuges eines Patienten und den erfindungsgemäßen Markersequenzen nachgewiesen wird. Eine solche Wechselwirkung ist z.B. eine Bindung, insbesondere eine bindende Substanz an mindestens einer erfindungsgemäßen Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

In einer weiteren Ausführungsform der Erfindung können jedoch die erfindungsgemäßen Markersequenzen in einer signifikant höheren oder niedrigeren Expressionsrate oder Konzentration vorliegen, dass auf die Prostataentzündungserkrankungen, Prostatakarzinom hinweist. Hierbei wird mittels Proteomics oder Nukleinsäureblots die relativen Expressionsraten krank / gesund der erfindungsgemäßen Markersequenzen für Prostataentzündungserkrankungen, Prostatakarzinom bestimmt.

Die Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen sind Gegenstand der Tabelle A und können durch den jeweilig zitierten Datenbankeintrag (auch mittels Internet: http://www.ncbi.nlm.nih.gov/) eindeutig identifiziert werden (siehe in Tabelle A: dort Accession No.), siehe ebenfalls das zugehörige Sequenzprotokoll.

Daher betrifft die Erfindung ebenfalls die Volllängesequenzen der erfindungsgemäßen Marker und zwar wie in Tabelle 1 über den bekannten Datenbankeintrag gemäß Tabelle A definiert, nachstehend SEQ la-174a genannt.

Weiterhin umfasst sind daher ebenfalls analoge Ausführungsformen von SEQ la-174a zu den Markersequenzen SEQ 1-174, wie z.B. in den Ansprüchen dargelegt, da die erfindungsgemäßen SEQ 1-174 wiederum Teilsequenzen, zumindest mit hoher Homologie, darstellen. Die spezifischen Markersequenzen SEQ 1-174 sind jedoch erfindungsgemäß bevorzugt.

Weiterhin bevorzugt sind SEQ 136a, 40a, 127a, 83a, 16a, 82a, 88a, 152a, 130a, 138a, 2a, 12a, 113a, 20a, 173a, 33a, 172a, 52a, 43a, 91a, 1a, 32a, 86a, 27a, 105a.

Erfindungsgemäß umfassen die Markersequenzen auch solche Modifikationen der cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

In einer weiteren Ausführungsform der Erfindung sind ebenfalls Teilsequenzen oder Fragmente der erfindungsgemäßen Markersequenzen umfasst. Insbesondere solche Teilsequenzen, die eine Identität von 95%, 90 %, insbesondere 80% oder 70 % mit den erfindungsgemäßen Markersequenzen aufweisen.

Teilsequenzen sind ebenfalls solche Sequenzen, die 50 bis 100 Nukleotide, 70-120 Nukleotide einer Sequenz der SEQ 1-174 aufweisen, oder davon erhältliche Peptide.

In einer weiteren Ausführungsform kann die jeweilige Markersequenz in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Ein weiterer Gegenstand der Erfindung betrifft eine Anordnung von Markersequenzen enthaltend mindestens eine Markersequenz einer cDNA ausgewählt aus der Gruppe SEQ 1 - 174 oder jeweils ein dafür kodierendes Protein. Vorzugsweise enthält die Anordnung mindestens 2 bis 5 oder 10, vorzugsweise 30 bis 50 Markersequenzen oder 50 bis 100 oder mehr Markersequenzen.

Im Rahmen dieser Erfindung bedeutet "Anordnung" synonym "Array" und sofern dieser "Array" zur Identifizierung von Substanzen an Markersequenzen verwendet wird, ist hierunter ein "Assay" oder eine diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Proteinbindern erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren. Ein Proteinbiochip im Sinne dieser Erfindung ist die systematische Anordnung von Proteinen auf einem festen Träger.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip bestehend aus einer Anordnung enthaltend erfindungsgemäße Markersequenzen.

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden. Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einen festen Träger fixiert, gespottet oder immobilisiert.

Daher betrifft die Erfindung eine Anordnung, wobei die Markersequenzen als Clone vorliegen.

Zusätzlich können die Markersequenzen in der jeweiligen Form in Form eines Fusionsproteins vorliegen, welches beispielsweise mindestens ein Affinitätsepiptop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz für Prostataentzündungserkrankungen, Prostatakarzinom, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Ferner betrifft die Erfindung ein Verfahren zum Identifizieren und Charakterisieren einer Substanz für Prostataentzündungserkrankungen, Prostatakarzinom, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Die zu untersuchende Substanz kann ein beliebiges natives oder nicht-natives Biomolekül, ein synthetisches chemisches Molekül, eine Mischung oder eine Substanzbibliothek sein.

Nachdem die zu untersuchende Substanz eine Markersequenz kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Prostataentzündungserkrankungen, Prostatakarzinom entwickelt und erhältlich durch den Einsatz des erfindungsgemäßen Assays oder Proteinbiochip.

Daher betrifft die Erfindung ebenfalls die Verwendung einer erfindungsgemäßen Anordnung oder einem Assay zum Screenen von Wirkstoffen für Prostataentzündungserkrankungen, Prostatakarzinom.

Daher betrifft die Erfindung in einer weiteren Ausführungsform ebenfalls ein Target zur Behandlung und Therapie von Prostataentzündungserkrankungen, Prostatakarzinom, jeweils ausgewählt aus der Gruppe SEQ 1 - 174 oder jeweils ein dafür kodierendes Protein.

In einer weiteren Ausführungsform betrifft die Erfindung ebenfalls die Verwendung der erfindungsgemäßen Markersequenzen, vorzugsweise in Form einer Anordnung, als Affinitätsmaterial zur Durchführung einer Apherese bzw. iwS. einer Blutwäsche, wobei Substanzen aus Körperflüssigkeiten eines Patienten mit Prostataentzündungserkrankungen, Prostatakarzinom, wie Blut oder Plasma, an die erfindungsgemäßen Markersequenzen binden und folglich der Körperflüssigkeit selektiv entzogen werden können.

### Beispiele und Figuren:

Zehn oder mehr Patientenproben wurden individuell gegen eine cDNA Expressionsbibliothek gescreent. Die Prostataentzündungserkrankungen, Prostatakarzinom - spezifischen Expressionsklone wurden ermittelt durch einen Vergleich mit zehn oder mehr gesunden Proben. Die Identität der Markersequenzen wurde durch DNA-Sequenzierung ermittelt.

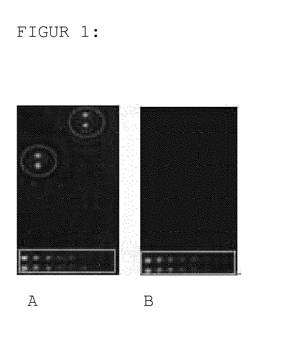

In Figur 1 wird das differentielle Screenen zwischen zwei Proteinbiochips aus jeweils einer cDNA-Expressionsbank eines Patienten und einem gesunden Probanden gezeigt. Die differentiellen Clone werden mittels Fluoresenzmarkierung nachgewiesen und bioinformatorisch ausgewertet.

Im Rahmen der Biomarkeridentifizierung werden verschiedene bioinformatische Analysen durchgeführt. Für jedes Serum werden mittels Microarray Reaktivitäten gegen ca. 2000 unterschiedliche Antigene gemessen. Diese Daten werden für ein Ranking der gespotteten Antigene bzgl. ihrer Differenzierungsfähigkeit zwischen gesunden und erkrankten Seren benutzt. Diese Auswertung wird mittels des nicht parametrischen Mann-Whitney Tests auf normalisierten Intensitätsdaten durchgeführt. Zur Normalisierung wird ein interner Standard benutzt, der auf jedem Chip mitgespottet wird. Da für jedes Antigen ein p-Wert berechnet wird, werden Methoden zur Korrektur des multiples Testens eingesetzt. Als sehr konservativer Ansatz wird eine Bonferroni Korrektur durchgeführt und zusätzlich wird die weniger restriktive False Discovery Rate (FDR) nach Benjamini & Hochberg berechnet. Desweiteren werden die Daten zur Klassifikation der Seren benutzt. Hierbei kommen unterschiedliche multivariate Methoden zum Einsatz. Dies sind Methoden aus den statistischen Lernverfahren wie Support Vector Machines (SVM), Neuronale Netze oder Klassifikationsbäume, sowie eine Schwellenwertmethode, welche sowohl zur Klassifikation als auch zur visuellen Repräsentation der Daten geeignet ist.

Zur Vermeidung von Overfitting wird eine 10fache Cross-Validierung der Daten durchgeführt.

**Tabelle A:**

| seq | **Accession** | Blast | Klone |
|---|---|---|---|
| 1a | gi\|113402448 | PREDICTED: Homo sapiens similar to CXYorf1-related protein (LOC653635), mRNA | 00800_578_N18 |
| 2a | gi\|113413768 | PREDICTED: Homo sapiens family with sequence similarity 59, member B (FAM59B), mRNA | 00800_570_014 |
| 3a | gi\|113414262 | PREDICTED: Homo sapiens SPEG complex locus (SPEG), mRNA | 00800_557_N13 |
| 4a | gi\|113418314 | PREDICTED: Homo sapiens NHS-like 1, transcript variant 5 (NHSL1), mRNA | 00800_507_H03 |
| 5a | gi\|113425012 | PREDICTED: Homo sapiens kinesin family member 26A (KIF26A), mRNA | 00800_583_H21 |
| 6a | gi\|113426606 | PREDICTED: Homo sapiens hypothetical protein LOC727910 (LOC727910), mRNA | 00800_550_A18 |
| 7a | gi\|113427260 | PREDICTED: Homo sapiens jumonji domain containing 3, transcript variant 3 (J MJ D3), mRNA | 00800_569_A13 |
| 8a | gi\|113428505 | PREDICTED: Homo sapiens widely-interspaced zinc finger motifs, transcript variant 10 (WIZ), mRNA | 00800_588_F10 |
| 9a | gi\|113428756 | PREDICTED: Homo sapiens zinc finger protein 154 (pHZ-92) (ZNF154), mRNA | 00800_597_K23 |
| 10a | gi\|113429538 | PREDICTED: Homo sapiens tetratricopeptide repeat domain 28 (TTC28), mRNA | 00800_556_D03 |
| 11 | gi\|113431093 | PREDICTED: Homo sapiens GIY-YIG domain containing 2, transcript variant 1 (GIYD2), mRNA | 00800_514_H03 |
| 12a | gi\|12751496 | Homo sapiens chromosome 8 open reading frame 33 (C8orf33), mRNA | 00800_601_K04 |
| 13a | gi\|13325056 | Homo sapiens solute carrier family 27 (fatty acid transporter), member 5 (SLC27A5), mRNA | 00800_520_A13 |
| 14a | gi\|13325058 | Homo sapiens ARP1 actin-related protein 1 homolog A, centractin alpha (yeast) (ACTR1A), mRNA | 00800_582_L08 |
| 15a | gi\|13375663 | Homo sapiens family with sequence similarity 77, member C (FAM77C), mRNA | 00800_586_F01 |
| 16a | gi\|13375724 | Homo sapiens chromosome 14 open reading frame 138 (C14orf138), mRNA | 00800_528_C03 |
| 17a | gi\|13904863 | Homo sapiens cytochrome P450, family 27, subfamily A, polypeptide 1 (CYP27A1), nuclear gene encoding mitochondrial protein, mRNA | 00800_525_H24 |
| 18a | gi\|14042967 | Homo sapiens spinster (SPIN1), mRNA | 00800_531_P04 |
| 19a | gi\|14110410 | Homo sapiens heterogeneous nuclear ribonucleoprotein D-like (HNRPDL), transcript variant 1, mRNA | 00800_520_A20 |
| 20a | gi\|14251213 | Homo sapiens DEAD (Asp-Glu-Ala-Asp) box polypeptide 24 (DDX24), mRNA | 00800_592_F13 |
| 21a | gi\|14591916 | Homo sapiens ribosomal protein S25 (RPS25), mRNA | 00800_553_C23 |
| 22a | gi\|14772189 | Homo sapiens chromosome 20 genomic contig, reference assembly | 00800_530_J21 |
| 23a | gi\|15431299 | Homo sapiens ribosomal protein L18a (RPL18A), mRNA | 00800_564_D11 |
| 24a | gi\|16905511 | Homo sapiens ribosomal protein, large, P1 (RPLP1), transcript variant 1, m RNA | 00800_530_C03 |
| 25a | gi\|17149837 | Homo sapiens FK506 binding protein 1A, 12kDa (FKBP1A), transcript variant 12B, mRNA | 00800_598_J17 |
| 26a | gi\|18390348 | Homo sapiens ribosomal protein L7a (RPL7A), mRNA | 00800_528_A14 |
| 27a | gi\|19743568 | Homo sapiens TRAF family member-associated NFKB activator (TANK), transcript variant 1, mRNA | 00800_541_P08 |
| 28a | gi\|20357526 | Homo sapiens guanine nucleotide binding protein (G protein), beta polypeptide 1 (GNB1), mRNA | 00800_583_H15 |
| 29a | gi\|21071045 | Homo sapiens SWI/SNF related, matrix associated, actin dependent regulator of chromatin, subfamily a, member 1 (SMARCA1), transcript variant 2, m RNA | 00800_588_J12 |
| 30a | gi\|21361156 | Homo sapiens homer homolog 3 (Drosophila) (HOMER3), mRNA | 00800_518_O10 |
| 31a | gi\|21389314 | Homo sapiens solute carrier family 25 (mitochondrial carrier; citrate transporter), member 1 (SLC25A1), mRNA | 00800_583_B14 |
| 32a | gi\|22027484 | Homo sapiens RAS, dexamethasone-induced 1 (RASD1), mRNA | 00800_564_E02 |
| 33a | gi\|22035555 | Homo sapiens BRF1 homolog, subunit of RNA polymerase III transcription initiation factor IIIB (S. cerevisiae) (BRF1), transcript variant 1, mRNA | 00800_525_C01 |
| 34a | gi\|22095372 | Homo sapiens LIM domain containing 2 (LIMD2), mRNA | 00800_568_D15 |
| 35a | gi\|22202623 | Homo sapiens glutathione transferase zeta 1 (maleylacetoacetate isomerase) (GSTZ1), transcript variant 1, mRNA | 00800_547_A15 |
| 36a | gi\|22212935 | Homo sapiens opioid receptor, sigma 1 (OPRS1), transcript variant 3, m RNA | 00800_523_E04 |
| 37a | gi\|22538452 | Homo sapiens phosphatidylinositol glycan anchor biosynthesis, class Q (PIGQ), transcript variant 1, mRNA | 00800_574_K18 |
| 38a | gi\|23111017 | Homo sapiens RNA binding motif protein 10 (RBM10), transcript variant 2, m RNA | 00800_586_M11 |
| 39a | gi\|23238227 | Homo sapiens carbohydrate (N-acetylglucosamine 6-O) sulfotransferase 7 (CHST7), mRNA | 00800_540_C04 |
| 40a | gi\|23308566 | Homo sapiens asparaginase like 1 (ASRGL1), mRNA | 00800_563_O13 |
| 41a | gi\|24234719 | Homo sapiens DnaJ (Hsp40) homolog, subfamily B, member 6 (DNAJB6), transcript variant 2, mRNA | 00800_528_M21 |
| 42a | gi\|24308032 | Homo sapiens formin binding protein 4 (FNBP4), mRNA | 00800_520_D08 |
| 43a | gi\|24308256 | Homo sapiens KIAA1576 protein (KIAA1576), mRNA | 00800_590_P16 |
| 44a | gi\|24475884 | Homo sapiens Ras association (RaIGDS/AF-6) domain family 7 (RASSF7), mRNA | 00800_512_C23 |
| 45a | gi\|27886683 | Homo sapiens Kv channel interacting protein 1 (KCNIP1), transcript variant 2, m RNA | 00800_585_M21 |
| 46a | gi\|28178831 | Homo sapiens isocitrate dehydrogenase 2 (NADP+), mitochondrial (IDH2), mRNA | 00800_532_L07 |
| 47a | gi\|28269671 | Homo sapiens serologically defined colon cancer antigen 8 (SDCCAGB), mRNA | 00800_589_C17 |
| 48a | gi\|28872795 | Homo sapiens CCAAT/enhancer binding protein (C/EBP), beta (CEBPB), mRNA | 00800_599_K11 |
| 49a | gi\|29800963 | Homo sapiens chromosome 10 genomic contig, reference assembly | 00800_584_I24 |
| 50a | gi\|29826322 | Homo sapiens adducin 1 (alpha) (ADD1), transcript variant 3, mRNA | 00800_602_C16 |
| 51a | gi\|29826324 | Homo sapiens adducin 1 (alpha) (ADD1), transcript variant 4, mRNA | 00800_506_H06 |
| 52a | gi\|30089990 | Homo sapiens acid phosphatase 1, soluble (ACP1), transcript variant 3, m RNA | 00800_601_N08 |
| 53a | gi\|30795226 | Homo sapiens histidyl-tRNA synthetase 2 (HARS2), mRNA | 00800_578_C22 |
| 54a | gi\|31083149 | Homo sapiens axin 1 (AXIN1), transcript variant 1, mRNA | 00800_555_A23 |
| 55a | gi\|31341380 | Homo sapiens sterile alpha motif domain containing 14 (SAMD14), mRNA | 00800_530_E15 |
| 56a | gi\|31377576 | Homo sapiens chromosome 10 open reading frame 13 (C10orf13), mRNA | 00800_533_B02 |
| 57a | gi\|31543618 | Homo sapiens splicing factor, arginine/serine-rich 1 (splicing factor 2, alternate splicing factor) (SFRS1), mRNA | 00800_512_M22 |
| 58a | gi\|31982913 | Homo sapiens WD repeat domain 54 (WDR54), mRNA | 00800_590_J15 |
| 59a | gi\|32171243 | Homo sapiens hypothetical protein DKFZp434G156 (NAG6), mRNA | 00800_537_L05 |
| 60a | gi\|32261293 | Homo sapiens protein kinase, interferon-inducible double stranded RNA dependent activator (PRKRA), mRNA | 00800_522_G17 |
| 61a | gi\|32454740 | Homo sapiens serpin peptidase inhibitor, clade H (heat shock protein 47), member 1, (collagen binding protein 1) (SERPINH1), mRNA | 00800_524_I10 |
| 62a | gi\|32490571 | Homo sapiens erythrocyte membrane protein band 4.1-like 3 (EPB41 L3), mRNA | 00800_567_M08 |
| 63a | gi\|33469963 | Homo sapiens splicing factor 4 (SF4), mRNA | 00800_518_L01 |
| 64a | gi\|33469975 | Homo sapiens activating transcription factor 4 (tax-responsive enhancer element B67) (ATF4), transcript variant 1, mRNA | 00800_570_D17 |
| 65a | gi\|33469983 | Homo sapiens protein disulfide isomerase family A, member 4 (PDIA4), mRNA | 00800_600_D18 |
| 66a | gi\|33598947 | Homo sapiens phospholipase C, gamma 1 (PLCG1), transcript variant 1, m RNA | 00800_536_F02 |
| 67a | gi\|34147350 | Homo sapiens RAS-like, family 11, member B (RASL11 B), mRNA | 00800_601_M15 |
| 68a | gi\|34147700 | Homo sapiens dehydrogenase/reductase (SDR family) member 13 (DHRS13), mRNA | 00800_578_D10 |
| 69a | gi\|34222379 | Homo sapiens family with sequence similarity 100, member B (FAM100B), mRNA | 00800_594_J07 |
| 70a | gi\|34452731 | Homo sapiens phosphatidylinositol 3,4,5-trisphosphate-dependent RAC exchanger 1 (PREX1), mRNA | 00800_530_E24 |
| 71a | gi\|37550981 | Homo sapiens chromosome 10 genomic contig, reference assembly | 00800_596_A22 |
| 72a | gi\|37551026 | Homo sapiens chromosome 10 genomic contig, reference assembly | 00800_540_H15 |
| 73a | gi\|38372936 | Homo sapiens chromatin modifying protein 2A (CHMP2A), transcript variant 1, m RNA | 00800_533_P22 |
| 74a | gi\|38372939 | Homo sapiens alpha-2-glycoprotein 1, zinc (AZGP1), mRNA | 00800_519_J13 |
| 75a | gi\|38524584 | Homo sapiens NADH dehydrogenase (ubiquinone) Fe-S protein 7, 20kDa (NADH-coenzyme Q reductase) (NDUFS7), mRNA | 00800_541_B07 |
| 76a | gi\|38569414 | Homo sapiens amyloid beta (A4) precursor protein-binding, family A, member 2 binding protein (APBA2BP), transcript variant 2, m RNA | 00800_582_B04 |
| 77a | gi\|38679885 | Homo sapiens splA/ryanodine receptor domain and SOCS box containing 3 (SPSB3), mRNA | 00800_509_O10 |
| 78a | gi\|38679891 | Homo sapiens protein (peptidylprolyl cis/trans isomerase) NIMA-interacting, 4 (parvulin) (PIN4), mRNA | 00800_579_A06 |
| 79a | gi\|38679903 | Homo sapiens ADP-ribosylation factor-like 8A (ARL8A), mRNA | 00800_562_N23 |
| 80a | gi\|38683848 | Homo sapiens fibroblast growth factor (acidic) intracellular binding protein (FIBP), transcript variant 1, mRNA | 00800_527_J24 |
| 81a | gi\|38788107 | Homo sapiens small glutamine-rich tetratricopeptide repeat (TPR)-containing, alpha (SGTA), mRNA | 00800_577_P08 |
| 82a | gi\|40354199 | Homo sapiens TPX2, microtubule-associated, homolog (Xenopus laevis) (TPX2), mRNA | 00800_541_F11 |
| 83a | gi\|40789263 | Homo sapiens hypothetical protein MGC11257 (MGC11257), mRNA | 00800_511_M24 |
| 84a | gi\|40795666 | Homo sapiens ubiquitin specific peptidase 4 (proto-oncogene) (USP4), transcript variant 2, mRNA | 00800_562_E18 |
| 85a | gi\|40805842 | Homo sapiens p300/CBP-associated factor (PCAF), mRNA | 00800_578_M10 |
| 86a | gi\|41352062 | Homo sapiens phosphofructokinase, platelet (PFKP), mRNA | 00800_548_E23 |
| 87a | gi\|41352714 | Homo sapiens vacuolar protein sorting 35 (yeast) (VPS35), mRNA | 00800_586_A05 |
| 88a | gi\|41393564 | Homo sapiens inositol 1,3,4-triphosphate 5/6 kinase (ITPK1), mRNA | 00800_578_K17 |
| 89a | gi\|41406095 | Homo sapiens DEAH (Asp-Glu-Ala-His) box polypeptide 38 (DHX38), mRNA | 00800_586_C18 |
| 90a | gi\|42734426 | Homo sapiens NGFI-A binding protein 2 (EGR1 binding protein 2) (NAB2), mRNA | 00800_570_C19 |
| 91a | gi\|44917603 | Homo sapiens SLIT-ROBO Rho GTPase activating protein 1 (SRGAP1), mRNA | 00800_574_I17 |
| 92a | gi\|4504618 | Homo sapiens insulin-like growth factor binding protein 7 (IGFBP7), mRNA | 00800_524_E19 |
| 93a | gi\|4505324 | Homo sapiens Sjogren's syndrome nuclear autoantigen 1 (SSNA1), mRNA | 00800_541_N09 |
| 94a | gi\|4507126 | Homo sapiens small nuclear ribonucleoprotein polypeptide C (SNRPC), mRNA | 00800_529_022 |
| 95a | gi\|45439358 | Homo sapiens triple functional domain (PTPRF interacting) (TRIO), mRNA | 00800_546 _I15 |
| 96a | gi\|4557766 | Homo sapiens methylmalonyl Coenzyme A mutase (MUT), nuclear gene encoding mitochondrial protein, mRNA | 00800_520_O01 |
| 97a | gi\|4557788 | Homo sapiens Norrie disease (pseudoglioma) (NDP), mRNA | 00800_598_K21 |
| 98a | gi\|45597176 | Homo sapiens TBC1 domain family, member 9B (with GRAM domain) (TBC1 D9B), transcript variant 2, mRNA | 00800_549_J10 |
| 99a | gi\|46198303 | Homo sapiens coiled-coil-helix-coiled-coil-helix domain containing 8 (CHCHD8), mRNA | 00800_601_M20 |
| 100a | gi\|46370090 | Homo sapiens chromosome 11 open reading frame 31 (C11 orf31), m RNA | 00800_586_C20 |
| 101a | gi\|46411160 | Homo sapiens aconitase 2, mitochondrial (ACO2), nuclear gene encoding mitochondrial protein, mRNA | 00800_578_O19 |
| 102a | gi\|47132573 | Homo sapiens protein kinase, AMP-activated, gamma 1 non-catalytic subunit (PRKAG1), transcript variant 1, mRNA | 00800_583_I09 |
| 103a | gi\|47132588 | Homo sapiens protein kinase N1 (PKN1), transcript variant 2, mRNA | 00800_585_K02 |
| 104a | gi\|4757793 | Homo sapiens acetylserotonin O-methyltransferase-like (ASMTL), mRNA | 00800_566_K12 |
| 105a | gi\|47717133 | Homo sapiens CDC-like kinase 2 (CLK2), transcript variant 1, mRNA | 00800_539_C01 |
| 106a | gi\|47933338 | Homo sapiens RNA binding motif protein 15 (RBM15), mRNA | 00800_587_F09 |
| 107a | gi\|48527950 | Homo sapiens golgi associated, gamma adaptin ear containing, ARF binding protein 1 (GGA1), transcript variant 1, mRNA | 00800_595_F17 |
| 108a | gi\|48675816 | Homo sapiens hypothetical protein FLJ10154 (FLJ10154), mRNA | 00800_552_M12 |
| 109a | gi\|49355764 | Homo sapiens ELAV (embryonic lethal, abnormal vision, Drosophila)-like 3 (Hu antigen C) (ELAVL3), transcript variant 2, mRNA | 00800_506_024 |
| 110a | gi\|50053889 | Homo sapiens chromosome 14 open reading frame 131 (C14orf131), mRNA | 00800_545_A12 |
| 111a | gi\|5032030 | Homo sapiens RNA binding motif protein 5 (RBM5), mRNA | 00800_506_B06 |
| 112a | gi\|50345295 | Homo sapiens complement component 4B (Childo blood group) (C4B), mRNA | 00800_602_A21 |
| 113a | gi\|50878292 | Homo sapiens tripartite motif-containing 45 (TRIM45), mRNA | 00800_529_L23 |
| 114a | gi\|51464897 | Homo sapiens chromosome 5 genomic contig, reference assembly | 00800_516_H09 |
| 115a | gi\|51466739 | Homo sapiens chromosome 8 genomic contig, reference assembly | 00800_573_P03 |
| 116a | gi\|51467074 | Homo sapiens chromosome 8 genomic contig, reference assembly | 00800_579_H02 |
| 117a | gi\|51473102 | Homo sapiens chromosome 16 genomic contig, reference assembly | 00800_583_L05 |
| 118a | gi\|51473128 | Homo sapiens chromosome 16 genomic contig, reference assembly | 00800_600_C22 |
| 119a | gi\|51474257 | Homo sapiens chromosome 17 genomic contig, reference assembly | 00800_538_I06 |
| 120a | gi\|51475307 | Homo sapiens chromosome 21 genomic contig, reference assembly | 00800_597_N16 |
| 121a | gi\|52138581 | Homo sapiens pim-3 oncogene (PIM3), mRNA | 00800_586_G15 |
| 122a | gi\|52632376 | Homo sapiens melanoma antigen family D, 1 (MAGED1), transcript variant 2, m RNA | 00800_550_I19 |
| 123a | gi\|54111426 | Homo sapiens RAB11 family interacting protein 4 (class II) (RAB11FIP4), mRNA | 00800_578_P18 |
| 124a | gi\|55741844 | Homo sapiens valyl-tRNA synthetase like (VARSL), mRNA | 00800_596_F14 |
| 125a | gi\|55770883 | Homo sapiens ubiquitin associated domain containing 1 (UBADC1), mRNA | 00800_600_F14 |
| 126a | gi\|55925649 | Homo sapiens transcription elongation factor A (SII)-like 2 (TCEAL2), mRNA | 00800_541_G08 |
| 127a | gi\|56117827 | Homo sapiens speckle-type POZ protein (SPOP), transcript variant 3, m RNA | 00800_574_K08 |
| 128a | gi\|56117829 | Homo sapiens speckle-type POZ protein (SPOP), transcript variant 4, m RNA | 00800_584_M23 |
| 129a | gi\|57242791 | Homo sapiens adenomatosis polyposis coli 2 (APC2), mRNA | 00800_532_G10 |
| 130a | gi\|57617038 | Homo sapiens tubulin tyrosine ligase-like family, member 12 (TTLL12), mRNA | 00800_589_A07 |
| 131a | gi\|5802969 | Homo sapiens AFG3 ATPase family gene 3-like 2 (yeast) (AFG3L2), nuclear gene encoding mitochondrial protein, mRNA | 00800_529 K21 |
| 132a | gi\|58530844 | Homo sapiens zyxin (ZYX), transcript variant 2, mRNA | 00800_546_G19 |
| 133a | gi\|5902121 | Homo sapiens spectrin, beta, non-erythrocytic 2 (SPTBN2), mRNA | 00800_541_H20 |
| 134a | gi\|5902157 | Homo sapiens ring finger protein 113A (RNF113A), mRNA | 00800_525_E17 |
| 135a | gi\|60498971 | Homo sapiens 3-phosphoinositide dependent protein kinase-1 (PDPK1), transcript variant 1, mRNA | 00800_584_D17 |
| 136a | gi\|61102726 | Homo sapiens La ribonucleoprotein domain family, member 1 (LARP1), transcript variant 1, mRNA | 00800_556_H18 |
| 137a | gi\|62750346 | Homo sapiens histone deacetylase 5 (HDAC5), transcript variant 1, mRNA | 00800_550_B21 |
| 138a | gi\|63082031 | Homo sapiens p53-associated parkin-like cytoplasmic protein (PARC), mRNA | 00800_526_A18 |
| 139a | gi\|63252907 | Homo sapiens IQ motif and WD repeats 1 (IQWD1), transcript variant 1, m RNA | 00800_506_F21 |
| 140a | gi\|63497678 | Homo sapiens chromosome 1 open reading frame 131 (C1orf131), mRNA | 00800_520_P24 |
| 141a | gi\|65301138 | Homo sapiens ATPase, Class II, type 9A (ATP9A), mRNA | 00800_582_O11 |
| 142a | gi\|65787264 | Homo sapiens lipopolysaccharide-induced TNF factor (LITAF), mRNA | 00800_508_D10 |
| 143a | gi\|66346709 | Homo sapiens membrane associated guanylate kinase, WW and PDZ domain containing 2 (MAGI2), mRNA | 00800_545_I24 |
| 144a | gi\|66348107 | Homo sapiens zinc finger protein 12 (ZNF12), mRNA | 00800_581_L20 |
| 145a | gi\|66879658 | Homo sapiens ADP-ribosylation factor 1 (ARF1), transcript variant 4, mRNA | 00800_552_O19 |
| 146a | gi\|6912325 | Homo sapiens family with sequence similarity 50, member B (FAM50B), mRNA | 00800_564_D04 |
| 147a | gi\|70609888 | Homo sapiens ribosomal protein S3A (RPS3A), mRNA | 00800_550_007 |
| 148a | gi\|7262387 | Homo sapiens asparaginyl-tRNA synthetase (NARS), mRNA | 00800_528_H03 |
| 149a | gi\|74027246 | Homo sapiens polyglutamine binding protein 1 (PQBP1), transcript variant 2, m RNA | 00800_591_P06 |
| 150a | gi\|7657670 | Homo sapiens upstream binding transcription factor, RNA polymerase I (UBTF), mRNA | 00800_530_G08 |
| 151a | gi\|7669552 | Homo sapiens valosin-containing protein (VCP), mRNA | 00800_533_A21 |
| 152a | gi\|7705400 | Homo sapiens HDCMA18P protein (HDCMA18P), mRNA | 00800_518_D22 |
| 153a | gi\|7706556 | Homo sapiens chromosome 9 open reading frame 78 (C9orf78), transcript variant 2, m RNA | 00800_562_K11 |
| 154a | gi\|77628146 | Homo sapiens endoplasmic reticulum protein 29 (ERP29), transcript variant 1, m RNA | 00800_580_J04 |
| 155a | gi\|77917603 | Homo sapiens ubiquitin-binding protein homolog (UBPH), mRNA | 00800_569_P20 |
| 156a | gi\|8051607 | Homo sapiens heme oxygenase (decycling) 2 (HMOX2), mRNA | 00800_528_O17 |
| 157a | gi\|83716023 | Homo sapiens kinesin family member 21 B (KIF21 B), mRNA | 00800_528_P18 |
| 158a | gi\|83776595 | Homo sapiens CaM kinase-like vesicle-associated (CAMKV), mRNA | 00800_512_F08 |
| 159a | gi\|87578395 | Homo sapiens microtubule-associated protein 2 (MAP2), transcript variant 1, m RNA | 00800_580_L07 |
| 160a | gi\|88942318 | Homo sapiens chromosome 1 genomic contig, reference assembly | 00800_568_K22 |
| 161a | gi\|88942921 | Homo sapiens chromosome 1 genomic contig, reference assembly | 00800_523_H16 |
| 162a | gi\|88955854 | Homo sapiens chromosome 2 genomic contig, alternate assembly (based on Celera assembly) | 00800_540_D18 |
| 163a | gi\|88999178 | Homo sapiens chromosome 6 genomic contig, alternate assembly (based on Celera assembly) | 00800_544_J14 |
| 164a | gi\|88999564 | Homo sapiens chromosome 6 genomic contig, alternate assembly (based on Celera assembly) | 00800_587_F02 |
| 165a | gi\|89028628 | Homo sapiens chromosome 8 genomic contig, alternate assembly (based on Celera assembly) | 00800_603_N12 |
| 166a | gi\|89037929 | Homo sapiens chromosome 14 genomic contig, alternate assembly (based on Celera assembly) | 00800_581_N05 |
| 167a | gi\|89057698 | Homo sapiens chromosome 19 genomic contig, alternate assembly (based on Celera assembly) | 00800_566_M21 |
| 168a | gi\|89059606 | Homo sapiens chromosome X genomic contig, reference assembly | 00800_516_G22 |
| 169a | gi\|89060486 | Homo sapiens chromosome X genomic contig, reference assembly | 00800_538_J13 |
| 170a | gi\|8922357 | Homo sapiens PRP38 pre-mRNA processing factor 38 (yeast) domain containing B (PRPF38B), mRNA | 00800_580_H16 |
| 171a | gi\|90652860 | Homo sapiens protein tyrosine phosphatase, non-receptor type 5 (striatum-enriched) (PTPN5), transcript variant 3, mRNA | 00800_583_A01 |
| 172a | gi\|90903237 | Homo sapiens glutathione peroxidase 4 (phospholipid hydroperoxidase) (GPX4), transcript variant 2, mRNA | 00800_560_E13 |
| 173a | gi\|94536841 | Homo sapiens ribose 5-phosphate isomerase A (ribose 5-phosphate epimerase) (RPIA), mRNA | 00800_525_P07 |
| 174a | gi\|94538369 | Homo sapiens zuotin related factor 1 (ZRF1), mRNA | 00800_582_P15 |

## Patentansprüche

1. Verwendung der Markersequenzen zur Diagnose von Prostataentzündungserkrankungen, Prostatakarzinom, wobei mindestens eine Markersequenz einer cDNA ausgewählt aus der Gruppe SEQ 1 - 174 und / oder SEQ la-174a oder jeweils ein dafür kodierendes Protein oder jeweils einer Teilsequenz oder Fragment davon an oder zu einem zu untersuchenden Patienten bestimmt wird.

2. Verwendung der Markersequenzen zur Diagnose von Prostataentzündungserkrankungen, Prostatakarzinom nach Anspruch 1, **dadurch gekennzeichnet**, mindestens 2 bis 5 oder 10, vorzugsweise 30 bis 50 Markersequenzen oder 50 bis 100 oder mehr Markersequenzen an oder zu einem zu untersuchenden Patienten bestimmt wird.

3. Verwendung der Markersequenzen zur Diagnose von Prostataentzündungserkrankungen, Prostatakarzinom nach Anspruch 1 oder 2, **dadurch gekennzeichnet, dass** die Bestimmung mittels in-vitro Diagnose erfolgt.

4. Verwendung einer Markersequenz einer cDNA jeweils ausgewählt aus der Gruppe SEQ 1 - 174 und / oder SEQ la-174a oder jeweils ein dafür kodierendes Protein oder jeweils einer Teilsequenz oder Fragment davon als Diagnostikum.

5. Verwendung der Markersequenzen zur Diagnose von Prostataentzündungserkrankungen, Prostatakarzinom nach einem der vorhergehenden Ansprüche, **dadurch gekennzeichnet, dass** die Markersequenzen auf einem festen Träger aufgebracht werden, insbesondere einen Filter, eine Membran, ein magnetisches oder Fluorophormarkiertes Kügelchen, ein Silizium-Wafer, Glas, Metall, Kunststoff, ein Chip, ein massenspektrometrisches Target oder eine Matrix.

6. Verfahren zur Diagnose von Prostataentzündungserkrankungen, Prostatakarzinom, wobei
a.) mindestens eine Markersequenz einer cDNA ausgewählt aus der Gruppe SEQ 1 - 174 und / oder SEQ la-174a oder jeweils ein dafür kodierendes Protein oder jeweils einer Teilsequenz oder Fragment davon auf einem festen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

7. Verfahren zum Stratifizieren, insbesondere zur Risikostratifizierung, oder zur Therapiesteuerung eines Patienten mit Prostataentzündungserkrankungen, Prostatakarzinom, wobei mindestens eine Markersequenz einer cDNA ausgewählt aus der Gruppe SEQ 1 - 174 und / oder SEQ la-174a oder jeweils ein dafür kodierendes Protein oder jeweils einer Teilsequenz oder Fragment davon an einem zu untersuchenden Patienten bestimmt wird.

8. Verfahren nach Anspruch 7, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

9. Anordnung von Markersequenzen enthaltend mindestens eine Markersequenz einer cDNA ausgewählt aus der Gruppe SEQ 1 - 174 und / oder SEQ la-174a oder jeweils ein dafür kodierendes Protein.

10. Anordnung nach Anspruch 9, **dadurch gekennzeichnet, dass** mindestens 2 bis 5 oder 10, vorzugsweise 30 bis 50 Markersequenzen oder 50 bis 100 oder mehr Markersequenzen enthalten sind.

11. Anordnung nach Anspruch 9, **dadurch gekennzeichnet, dass** die Markersequenzen als Clone vorliegen.

12. Assay, Proteinbiochip bestehend aus einer Anordnung nach Anspruch 9, **dadurch gekennzeichnet, dass** die Markersequenzen auf einem festen Träger aufgebracht sind.

13. Verwendung einer Anordnung nach einem der Ansprüche 9 bis 11 oder einem Assay nach Anspruch 12 zum Identifizieren und Charakterisieren einer Substanz für Prostataentzündungserkrankungen, Prostatakarzinom enthaltend Mittel zum Nachweis eines Bindungserfolges, **dadurch gekennzeichnet, dass** eine Anordnung oder Assay mit a.) mindestens einer zu untersuchenden Substanz in Kontakt gebracht wird und b.) ein Bindungserfolg nachgewiesen wird.

14. Verwendung einer Anordnung nach einem der Ansprüche 9 bis 11 oder einem Assay nach Anspruch 12 zum Screenen von Wirkstoffen für Prostataentzündungserkrankungen, Prostatakarzinom.

15. Diagnostika zur Diagnose von Prostataentzündungserkrankungen, Prostatakarzinom, jeweils ausgewählt aus der Gruppe SEQ 1 - 174 und / oder SEQ la-174a oder jeweils ein dafür kodierendes Protein oder jeweils einer Teilsequenz oder Fragment davon.

16. Target zur Behandlung und Therapie von Prostataentzündungserkrankungen, Prostatakarzinom, jeweils ausgewählt aus der Gruppe SEQ 1 - 174 und / oder SEQ la-174a oder jeweils ein dafür kodierendes Protein oder jeweils einer Teilsequenz oder Fragment davon.

17. Verwendung einer Markersequenz einer cDNA ausgewählt aus der Gruppe SEQ 1 - 174 und / oder SEQ la-174a oder jeweils ein dafür kodierendes Protein oder jeweils einer Teilsequenz oder Fragment davon als Affinitätsmaterial zur Durchführung einer Apherese oder Blutwäsche für Patienten mit Prostataentzündungserkrankungen, Prostatakarzinom.


SEQUENCE LISTING

<110>  Protagen Aktiengesellschaft

<120>  Markersequenzen für Prostataentzündungserkrankungen,
       Prostatakarzinom und deren Verwendung

<130>  76WO 2008/01

<140>  PCT/DE2009/058534
<141>  2009-07-04

<160>  174

<170>  PatentIn version 3.3

<210>  1
<211>  255
<212>  DNA
<213>  Homo Sapiens

<400>  1
gaatcgtcga cccacgcgtt ccgctcgcag gcaccatgac tcctgtgagg atgcagcact      60

ccctggcagg tcagacctat gccgtgccct tcatccagcc agacctgcgg cgagaggagg     120

ccgtccagca gatggcggat gccctgcagt acctgcagaa ggtctctgga gacatcttca     180

gcaggtgttc tccagtgcca agtaccctgc tccagggcgc ctgcaggaat atggctccat     240

cttcacgggc gccca                                                      255


<210>  2
<211>  255
<212>  DNA
<213>  Homo sapiens

<400>  2
gaatcgtcga cccacgcgtc cgcctgtggc tacctctggc cctgcgtatt ccccaggccc      60

agcctcgcca ggccaggcct attcagctgc tcccccctcc tcctgcgccc cctcctcctc     120

ctcttcttct gaatggcagg aaccagtcct ggagcccttc gatccctttg agctggggca     180

gggcagttct ccagagcctg agctgctgcg ttctcaggag cccagagcag tggggacacc     240

tgggcctgga ccccg                                                      255


<210>  3
<211>  255
<212>  DNA
<213>  Homo sapiens

<400>  3
gaatcgtcga cccacgcgtc cgcctcccct gagctcgagt cttcggatga ctcctacgtg      60

tccgctggag aagagcccct agaggcccct gtgtttgaga tccccctgca gaatgtggtg     120

gtggcaccag gggcagatgt gctgctcaag tgtatcatca ctgccaaccc cccgccccaa     180

gtgtcctggc acaaggatgg gtcagcgctg cgcagcgagg gccgcctcct cctccgggct     240

gagggtgagc ggcac 255

<210> 4
<211> 255
<212> DNA
<213> Homo sapiens

<400> 4
gaatcgtcga cccacgcgtc cgcggacgcg tgggttgcag tggagcccgc agagaacgtg 60

agcgaagccc tccgagctgt gcccagcccc acgacgggag aggagggctc tgtgcacagc 120

agggaggcaa aagagagttc tgcagcccaa gctggctctc atgccacgca ccctggcacc 180

tcggttcttg agggaggagc tgcaggatcc atgtctccca gcagagtgga agccaatgtc 240

cccatggttc agcct 255

<210> 5
<211> 255
<212> DNA
<213> Homo sapiens

<400> 5
gaatcgtcga cccacgcgtc cggaagcggc acaccgtctc gccggacacg cgtctctgga 60

gcggtacgaa ggcctggcgc acagcagcag caagggccgg gaagcccctg ggcggcctcc 120

ccgggctgta cccaagctgg gtgtgccacc ctccagcccc acacacggtc cagctcccgc 180

ctgtaggagc ggcgcagcca aggctgtggg ggcccccaag ccccctgttg gtggaggcaa 240

gggccgtggc ctagt 255

<210> 6
<211> 255
<212> DNA
<213> Homo sapiens

<400> 6
gaatcgtcga cccacgcgtc cgaaaggcgc ggcggggagg ggagccgggg ccccagccct 60

gccgggaaac gggttcccag tcccggccga gagcgcgggc gccgagggga ccgacccgga 120

ctccccgcaa ccccccaacc gccaggccat ggcgcccacg gggctcctgg tggccggcgc 180

ctccttcctc gcgttccggg ggctgcactg ggggttgcgg cggctgccca cgccggaatc 240

ggccgctcgg gaccg 255

<210> 7
<211> 255
<212> DNA
<213> Homo sapiens

<400> 7
gaatcgtcga cccacgcgtc cgggctgcct ggaggcagat gctcagccag cattgggcag 60

cccccgcttc ctgctcccct acccccttca catggcagta gttctgggca ccccagcaaa 120

ccatattatg ctccaggggc gcccactcca agacccctcc atgggaagct ggaatccctg 180

catggctgtg tgcaggcatt gctccgggag ccagcccagc cagggctttg ggaacagctt 240

gggcaactgt acgag 255

<210> 8
<211> 255
<212> DNA
<213> Homo sapiens

<220>
<221> misc_feature
<222> (34)..(34)
<223> n is a, c, g, or t

<400> 8
gaatcgtcga cccacgcgtc cggacggacc cagntctcgg cctgggtgga cctcccaacc 60

caccagggcc aagcccaaaa gccctggcca agatgatggg cggcgcaggt cctggcagct 120

cactggaagc ccgcagcccc tcggaccttc acatctcacc cttggccaag aagttgccac 180

caccaccggg cagccccctg ggccactcac caactgcctc tcctcctcct acggcccgaa 240

agatgttccc aggcc 255

<210> 9
<211> 255
<212> DNA
<213> Homo sapiens

<400> 9
gaatcgtcga cccacgcgtc cgggtgtgtt caccggatca gagatagcag agcgccgagt 60

tggggccacg aaggcgtgag gggagtcgtc gtccctcctg cacgaaagcg tctaagcctt 120

ggcgacgccg ccctggggga cccacgtcag gcctgggata gggaccgctg tccccgggtc 180

cctaccaatg tcgcccgtcg ctcccggccc agctctaccc gcagagtctg atggcagcgg 240

ccactctgag gacgc 255

<210> 10
<211> 255
<212> DNA
<213> Homo sapiens

<400> 10
gaatcgtcga cccacgcgtc cgagctaccc aagcgcctca gccttgacag ctcctcctcc 60

ctcgagtctc ttgcttctgc tcagtctgtt tccaacgccc tgcccttggg ttaccagcaa 120

ccccccttct ctcccaccgg tgcggacagc atcgcctcag atgccatctc tgtgtacagt 180

ctgagctcca ttgcctcctc aatgagcttt gtctccaaac ccgagggtgg atcagagggt 240

ggaggccccg gagga                                                    255

<210>  11
<211>  255
<212>  DNA
<213>  Homo sapiens

<400>  11
gaatcgtcga cccacgcgtc cgggtcacgg caatcaaccc cctgctgtgg ttcccgaacc      60

ccaaggcccg atgggtcccg cgggggtcgc ggcgaggcca gggcgctttt tcggcgtcta     120

cctgctctac tgcctgaacc cccggtaccg gggccgcgtc tacgtggggt tcactgtcaa     180

cactgctcgt cgggtccagc agcacaatgg gggccgcaaa aaaggcgggg cctggcggac     240

cagcgggcga gggcc                                                    255

<210>  12
<211>  255
<212>  DNA
<213>  Homo sapiens

<400>  12
gaatcgtcga cccacgcgtc cgagaaaacg cggaacaggg cctctgtggc aaatggaggc      60

gagaaggcct cagagaaact cgccccagaa gaagttcccc taagcgctga ggcccaggca     120

caacagttgg cccaggaatt ggcttggtgt gtggagcaac tggagctggg cctcaagagg     180

cagaaaccca ccccgaaaca gaaagagcag gctattggag caatccgaac cctgcgcagc     240

aaaagaacgc ccttg                                                    255

<210>  13
<211>  182
<212>  DNA
<213>  Homo sapiens


<220>
<221>  misc_feature
<222>  (4)..(4)
<223>  n is a, c, g, or t

<220>
<221>  misc_feature
<222>  (29)..(29)
<223>  n is a, c, g, or t

<220>
<221>  misc_feature
<222>  (32)..(33)
<223>  n is a, c, g, or t

<220>
<221>  misc_feature
<222>  (37)..(37)
<223>  n is a, c, g, or t

<220>
<221>  misc_feature
<222>  (41)..(41)
<223>  n is a, c, g, or t

<220>
<221>  misc_feature
<222>  (44)..(44)
<223>  n is a, c, g, or t

<220>
<221>  misc_feature
<222>  (46)..(46)
<223>  n is a, c, g, or t

<220>
<221>  misc_feature
<222>  (49)..(49)
<223>  n is a, c, g, or t

<220>
<221>  misc_feature
<222>  (57)..(57)
<223>  n is a, c, g, or t

<220>
<221>  misc_feature
<222>  (68)..(68)
<223>  n is a, c, g, or t

<220>
<221>  misc_feature
<222>  (71)..(71)
<223>  n is a, c, g, or t

<220>
<221>  misc_feature
<222>  (115)..(115)
<223>  n is a, c, g, or t

<220>
<221>  misc_feature
<222>  (118)..(120)
<223>  n is a, c, g, or t

<220>
<221>  misc_feature
<222>  (133)..(133)
<223>  n is a, c, g, or t

<220>
<221>  misc_feature
<222>  (138)..(138)
<223>  n is a, c, g, or t

<220>
<221>  misc_feature
<222>  (168)..(168)
<223>  n is a, c, g, or t

<220>
<221>  misc_feature
<222>  (171)..(171)

<223>  n is a, c, g, or t

<220>
<221>  misc_feature
<222>  (177)..(177)
<223>  n is a, c, g, or t

<220>
<221>  misc_feature
<222>  (182)..(182)
<223>  n is a, c, g, or t

<400>  13
atanaatcgg tcgacccacg cgtccgggna gnntgtnagc ngtngncgnc tgccaanatg      60

ccaccccngg ngtcgggttg cgccatgctg cgagcagcga gtgagcctgt accanccnnn     120

ctctgtagtc ttntgagnag gattccactc ctgtgtcctg tgcgtacnaa natgaancgg     180

tn                                                                    182


<210>  14
<211>  255
<212>  DNA
<213>  Homo sapiens

<400>  14
gaatcgtcga cccacgcgtc cgcccacctt tgcagatatc tgcacctcag gagagactgt      60

attccacgtg gattgggggc tccatccttg cctccctgga cacctttaag aagatgtggg     120

tctccaaaaa ggaatatgag gaagacggtg cccgatccat ccacagaaaa accttctaat     180

gtcgggacat catcttcacc tctctctgaa gttaactcca ctttaaaact cgctttcttg     240

agtcggagtg tttgc                                                      255


<210>  15
<211>  255
<212>  DNA
<213>  Homo sapiens

<400>  15
gaatcgtcga cccacgcgtc cgcccacgcg tccggatgga tgaagcccca aattcagccc      60

cacccacaga gaagccttcc tacactcagc ctctgtccac ccttggcaaa tctttcaagc     120

tctctcctcc aggaaagtgg ggccccaact cagtcactcc acccccttcc aggtccctga     180

ggctggttct actgtatccc catcacctcc acaactccac tcacccctga cggctccatc     240

cacctcacca gttgg                                                      255


<210>  16
<211>  255
<212>  DNA
<213>  Homo sapiens


<220>

<221>  misc_feature
<222>  (9)..(9)
<223>  n is a, c, g, or t

<220>
<221>  misc_feature
<222>  (16)..(16)
<223>  n is a, c, g, or t

<220>
<221>  misc_feature
<222>  (18)..(18)
<223>  n is a, c, g, or t

<220>
<221>  misc_feature
<222>  (22)..(22)
<223>  n is a, c, g, or t

<220>
<221>  misc_feature
<222>  (29)..(29)
<223>  n is a, c, g, or t

<220>
<221>  misc_feature
<222>  (36)..(36)
<223>  n is a, c, g, or t

<220>
<221>  misc_feature
<222>  (55)..(55)
<223>  n is a, c, g, or t

<220>
<221>  misc_feature
<222>  (60)..(60)
<223>  n is a, c, g, or t

<220>
<221>  misc_feature
<222>  (84)..(84)
<223>  n is a, c, g, or t

<220>
<221>  misc_feature
<222>  (103)..(103)
<223>  n is a, c, g, or t

<220>
<221>  misc_feature
<222>  (108)..(108)
<223>  n is a, c, g, or t

<220>
<221>  misc_feature
<222>  (118)..(118)
<223>  n is a, c, g, or t

<220>
<221>  misc_feature
<222>  (121)..(121)
<223>  n is a, c, g, or t

<220>
<221>  misc_feature
<222>  (125)..(125)
<223>  n is a, c, g, or t

<220>
<221>  misc_feature
<222>  (130)..(131)
<223>  n is a, c, g, or t

<220>
<221>  misc_feature
<222>  (140)..(140)
<223>  n is a, c, g, or t

<220>
<221>  misc_feature
<222>  (144)..(144)
<223>  n is a, c, g, or t

<220>
<221>  misc_feature
<222>  (159)..(159)
<223>  n is a, c, g, or t

<220>
<221>  misc_feature
<222>  (162)..(162)
<223>  n is a, c, g, or t

<220>
<221>  misc_feature
<222>  (172)..(172)
<223>  n is a, c, g, or t

<220>
<221>  misc_feature
<222>  (217)..(217)
<223>  n is a, c, g, or t

<220>
<221>  misc_feature
<222>  (238)..(238)
<223>  n is a, c, g, or t

<220>
<221>  misc_feature
<222>  (241)..(241)
<223>  n is a, c, g, or t

<220>
<221>  misc_feature
<222>  (244)..(244)
<223>  n is a, c, g, or t

<220>
<221>  misc_feature
<222>  (251)..(251)
<223>  n is a, c, g, or t

<400>  16
aaatcgtcna cccacncntc cncgatggng gatacnctgg agtccatcgc tggangaccn      60

acatgcggag catttgtgcg agtnttggag aagacgggat ggnacagntg ctaccacnac 120

ngcantatan ntccggtggn gtgngttgcg ttgtgtgcna cnctgccatt gncctttcta 180

aatacctgaa aaacgcccga gttttctggc gacgggnacc acgcgctgag ccggcggncg 240

ntgntggagc ngggt 255

<210> 17
<211> 255
<212> DNA
<213> Homo sapiens

<400> 17
gaatcgtcga cccacgcgtc cggcacaacc catggctgcg ctgggctgcg cgaggctgag 60

gtgggcgctg cgaggggccg gccgtggcct ctgcccccac ggggccagag ccaaggccgc 120

gatccctgcc gccctcccct cggacaaggc caccggagct cccggagccg ggcctggtgt 180

ccggcggcgg caacggagct tagaggagat tccacgtcta ggacagctgc gcttcttctt 240

tcagctgttc gttca 255

<210> 18
<211> 255
<212> DNA
<213> Homo sapiens

<400> 18
gaatcgtcga cccacgcgtc cgcggacgcg tgggcggacg cgtgggcgga cgcgtgggct 60

ggtgggggtc ggggaggcca gttattccac catcgcgccc actctcattg ccgacctctt 120

tgtggccgac cagcggagcc ggatgctcag catcttctac tttgccattc cggtgggcag 180

tggtctgggc tacattgcag gctccaaagt gaaggatatg gctggagact ggcactgggc 240

tctgagggtg acacc 255

<210> 19
<211> 255
<212> DNA
<213> Homo sapiens

<400> 19
gaatcgtcga cccacgcgtc cgccaagatc aacgcgagca agaatcagca ggatgacggt 60

aaaatgttta ttggaggctt gagctgggat acaagcaaaa aagatctgac agagtacttg 120

tctcgatttg gggaagttgt agactgcaca attaaaacag atccagtcac tgggagatca 180

agaggatttg gatttgtgct tttcaaagat gctgctagtg ttgataaggt tttggaactg 240

aaagaacaca aactg 255

<210> 20
<211> 255

<212>  DNA
<213>  Homo sapiens

<400>  20
gaatcgtcga cccacgcgtc cgaccgaagt ggtcgaactg ctcgagctac caatgaaggc      60

ctcagtctga tgctcattgg gcctgaggat gtgatcaact ttaagaagat ttacaaaacg     120

ctcaagaaag atgaggatat cccactgttc cccgtgcaga caaaatacat ggatgtggtc     180

aaggagcgaa tccgtttagc tcgacagatt gagaaatctg agtatcggaa cttccaggct     240

tgcctgcaca actct                                                      255


<210>  21
<211>  255
<212>  DNA
<213>  Homo Sapiens

<400>  21
gaatcgtcga cccacgcgtc cgcccacgcg tccggacgag gctgcggtgt ctgctgctat      60

tctccgagct tcgcaatgcc gcctaaggac gacaagaaga agaaggacgc tggaaagtcg     120

gccaagaaag acaaagaccc agtgaacaaa tccgggggca aggccaaaaa gaagaagtgg     180

tccaaaggca aagttcggga caagctcaat aacttagtct tgtttgacaa agctacctat     240

gataaactct gtaag                                                      255


<210>  22
<211>  255
<212>  DNA
<213>  Homo Sapiens

<400>  22
gaatcgtcga cccacgcgtc cgaggtgaga ggcgcctctg cccagctgcc cctactggga      60

agtgaggagc ccctctgccc ggctagccgc cccgtccagg agggaggtgg gggggtcagc     120

cccccaccca gccagccgcc ttgtccggga gggaggtggg gtgatcagcc ccccacctgg     180

ccagccaccc catccgggag ggaggtgggg tggttaaccc cccgccaggc cagccgcccc     240

ctccaggagg gaggt                                                      255


<210>  23
<211>  255
<212>  DNA
<213>  Homo Sapiens

<400>  23
gaatcgtcga cccacgcgtc cggcggcgaa cgcggagagc acgccatgaa ggcttcgggc      60

acgctacgag agtacaaggt agtgggtcgc tgcctgccca cccccaaatg ccacacgccg     120

cccctctacc gcatgcgaat ctttgcgcct aatcatgtcg tcgccaagtc ccgcttctgg     180

tactttgtat ctcagttaaa gaagatgaag aagtcttcag gggagattgt ctactgtggg     240

caggtgtttg agaag                                                    255

<210>  24
<211>  255
<212>  DNA
<213>  Homo Sapiens

<400>  24
gaatcgtcga cccacgcgtc cggggtgagg ccctcacttc atccggcgac tagcaccgcg      60

tccggcagcg ccagccctac actcgcccgc gccatggcct ctgtctccga gctcgcctgc     120

atctactcgg ccctcattct gcacgacgat gaggtgacag tcacggagga taagatcaat     180

gccctcatta aagcagccgg tgtaaatgtt gagccttttt ggcctggctt gtttgcaaag     240

gccctggcca acgtc                                                      255

<210>  25
<211>  255
<212>  DNA
<213>  Homo Sapiens

<400>  25
gaatcgtcga cccacgcgtc cgcccacgcg tccgaaccgc cgccaggtcg ctgttggtcc      60

acgccgcccg tcgcgccgcc cgcccgctca gcgtccgccg ccgccatggg agtgcaggtg     120

gaaaccatct ccccaggaga cgggcgcacc ttccccaagc gcggccagac ctgcgtggtg     180

cactacaccg ggatgcttga agatggaaag aaatttgatt cctcccggga cagaaacaag     240

ccctttaagt ttatg                                                      255

<210>  26
<211>  255
<212>  DNA
<213>  Homo Sapiens

<400>  26
gaatcgtcga cccacgcgtc cgcgccgccc aagatgccga aaggaaagaa ggccaaggga      60

aagaaggtgg ctccggcccc agctgtcgtg aagaagcagg aggctaagaa agtggtgaat     120

cccctgtttg agaaaaggcc taagaatttt ggcattggac aggacatcca gcccaaaaga     180

gacctcaccc gctttgtgaa atggccccgc tatatcaggt tgcagcggca gagagccatc     240

ctctataagc ggctg                                                      255

<210>  27
<211>  255
<212>  DNA
<213>  Homo Sapiens

<400>  27
gaatcgtcga cccacgcgtc cggaggtgca ccatccatca catctgtcac accaagagga      60

ctgtgcagag atgaggaaga cacctctttt gaatcacttt ctaaattcaa tgtcaagttt     120

ccacctatgg acaatgactc aactttctta catagcactc cagagagacc cggcatcctt 180

agtcctgcca cgtctgaggc agtgtgccaa gagaaattta atatggagtt cagagacaac 240

ccagggaact ttgtt 255

<210> 28
<211> 255
<212> DNA
<213> Homo Sapiens

<220>
<221> misc_feature
<222> (1)..(1)
<223> n is a, c, g, or t

<220>
<221> misc_feature
<222> (81)..(81)
<223> n is a, c, g, or t

<220>
<221> misc_feature
<222> (190)..(190)
<223> n is a, c, g, or t

<220>
<221> misc_feature
<222> (242)..(242)
<223> n is a, c, g, or t

<400> 28
naatcgtcga cccacgcgtc cggaggcggc ggcggcggcg gcgggagcgg gagcgggcgg 60

cgagtgggga gcggggccgg nagtggagca gccgccgcgg cgggactgga ccgagcctcg 120

ccggcgcgca cctgcccgca gcgcccgcgg agcgcgcagc gcggcccgag cgcgacgacc 180

tgccgagcgn cggccgaggc ggcggtgtgg gcgcgtcagg ccgcgacgag ggcgctgaga 240

cnaatttaca tgtat 255

<210> 29
<211> 255
<212> DNA
<213> Homo Sapiens

<400> 29
gaatcgtcga cccacgcgtc cgggaatcct gatatcccaa atccagctct ggctcaaaga 60

gaagagcaaa aaaagattga tggagctgaa cctcttacac cagaagagac tgaagaaaag 120

gaaaaacttc tcacacaagg tttcacaaac tggactaaac gagattttaa ccagtttatt 180

aaagctaatg agaaatatgg aagagatgac attgataaca tagctcgaga ggtagagggc 240

aaatcccctg aggag 255

<210>  30
<211>  255
<212>  DNA
<213>  Homo Sapiens

<400>  30
gaatcgtcga cccacgcgtc cgcccacgcg tccggcggcc gcgtcgggac cggccgcgat      60

gggaccggag gcggcgcggg cggcggcggc gcagacaaag gggccaaacc agtgctcctg     120

ccacctctct ggctgccccc tagagcctgc ccatcccagc ctgaccaatg tccacagcca     180

gggagcagcc aatcttcagc acacgggcgc acgtgttcca aattgaccca gccaccaagc     240

gaaactggat cccag                                                      255


<210>  31
<211>  255
<212>  DNA
<213>  Homo Sapiens

<400>  31
gaatcgtcga cccacgcgtc cggccccagg caccgccaga gtgtcctgct acctttgtct      60

cacgattcca gtgcagtagt gccaaaaggc cccttcccac gtccctcgag ctctgtagcc     120

tggtctgtgc attgtggctg tcaaatccat gtgtcccccc tgtggtcgtg tgccccatgg     180

agaccatcaa ggtgaagttc atccacgacc agacctcccc aaaccccaag tacagaggat     240

tcttccacgg ggtta                                                      255


<210>  32
<211>  255
<212>  DNA
<213>  Homo Sapiens

<400>  32
gaatcgtcga cccacgcgtc cggcctgtgc ccagatcctg ggagaacccc agccgagccc      60

agcctagccc gagcccagcc cgagcggagc cggagcccca agcccgagcc gcgcccagcc     120

cgagcagagc cctccagccg ctcaccccgc gtgccacccc agcgaccctc agccgctctc     180

tgcccttctc tcggccccgc gcccgccctc gcggcccctc tgcccaatga aactggccgc     240

gatgatcaag aagat                                                      255


<210>  33
<211>  255
<212>  DNA
<213>  Homo Sapiens

<400>  33
gaatcgtcga cccacgcgtc cgagcagctt gaacaagtcc tgtcaaaaaa actggaggag      60

gttgaaggtg aaatatccag ttaccaggat gcaattgaga ttgaactaga aaacagccgg     120

ccaaaggcca aggggggcct ggccagcctg gcaaaagatg gctccaccga ggacaccgcg     180

tccagcttgt gtggcgagga ggacacagag gacgaggagc tggaagccgc ggccagccac 240

ctgaacaaag actta 255

<210> 34
<211> 255
<212> DNA
<213> Homo Sapiens

<400> 34
gaatcgtcga cccacgcgtc cgcagacggc gcggccgaac ccagcgggtg ccgcttctcc 60

acccgaggct tccacctcca acgagccatg ttccaggctg caggagccgc ccaggccacc 120

ccctctcatg acgccaaagg cggcggcagc agcacggtgc agcgctccaa gtccttcagc 180

ctgcgggccc aggtgaagga gacctgcgcc gcctgccaga agaccgtgta ccccatggag 240

cggctggtgg ccgac 255

<210> 35
<211> 255
<212> DNA
<213> Homo Sapiens

<400> 35
gaatcgtcga cccacgcgtc cgggaggggg tcgcgcgaag tgccagatgc aggcggggaa 60

gcccatcctc tattcctatt tccgaagctc ctgctcatgg agagttcgaa ttgctctggc 120

cttgaaaggc atcgactacg agacggtgcc catcaatctc ataaaggatg ggggccaaca 180

gttttctaag gacttccagg cactgaatcc tatgaagcag gtgccaaccc tgaagattga 240

tggaatcacc attca 255

<210> 36
<211> 255
<212> DNA
<213> Homo Sapiens

<400> 36
gaatcgtcga cccacgcgtc cgggaagacc tgcggatgga caggagcggg caggcccgca 60

catatccact tgctggagcc catgtttaca gacagggaca tacaccatgc agatcctgag 120

ttcctgctgt atgagcaggg atatccatgc ttatgtatcc aaacacagag acccatggga 180

acaaatgaga cacatataga tactgagacc tgtgtgtaca gtaggaccat gcactcacac 240

ccatctggag aggga 255

<210> 37
<211> 255
<212> DNA
<213> Homo Sapiens

<400> 37
gaatcgtcga cccacgcgtc cgcccacgcg tccgcccacg cgtccggtgg aggccagcat 60

cctcgcggag ctggccaggc gagcctcggg acccatttgt ctgctgttgg ccagcctgct     120

gtcgctggtc tcagctgtca gtgcctgccg agtgttcaag ctctggcccc tgtccttcct     180

cgggagcaaa ctctccacgt gcgaacagct ccggcaccgg ctggagcacc tcacgctaat     240

cttcagtaca cggaa                                                      255


<210>  38
<211>  255
<212>  DNA
<213>  Homo Sapiens

<400>  38
gaatcgtcga cccacgcgtc cggttctaag agggacatgg cctccaatga aggcagtcgc      60

atcagtgctg cctctgtggc cagcactgcc attgctgcgg cccagtgggc catctcacag     120

gcctcccaag gtggggaggg tacctgggcc acctccgagg agccgccggt cgactacagc     180

tactaccaac aggatgaggg ctatggcaac agccagggca cagagtcttc cctctatgcc     240

catggctacc tcaag                                                      255


<210>  39
<211>  255
<212>  DNA
<213>  Homo Sapiens

<400>  39
gaatcgtcga cccacgcgtc cgggcctcaa cctgaaggtg gtgcagcttt tccgcgaccc      60

gagggcggtg cacaactcgc gcctcaagtc taggcaggga ctgctgcgcg agagcatcca     120

ggtgctgcgc acccgccaga ggggcgaccg cttccaccgt gtgctgctgg cgcacggcgt     180

gggtgctcgc cccgggggcc agtctcgcgc gctgcccgcc gcgccgcgcg ccgatttctt     240

cctgaccggt gcgct                                                      255


<210>  40
<211>  255
<212>  DNA
<213>  Homo Sapiens

<400>  40
gaatcgtcga cccacgcgtc cgcccacgcg tccgcccacg cgttccgccc acgcgtccgg      60

ccggcgtcgg ggagcggcgg taccgggcgg ctgcggggct ggctcgaccc agctggaggt     120

ctcggcgtcc gcgtcctgcg gtgccctggg acccgccgac atgaatccca tcgtagtggt     180

ccacggcggc ggagccggtc ccatctccaa ggatcggaag gagcgagtgc accagggcat     240

ggtcagagcc gccac                                                      255


<210>  41
<211>  255

<212>  DNA
<213>  Homo Sapiens

<400>  41
gaatcgtcga cccacgcgtc cggagagaaa ggaaagcgcg aggagccgcc gccaccacca      60

gcgcagcagt cctggagctg tgaggagatt cgggccgtca ccctgcctcc cctgcgtccc     120

gccaccggcc gcttctgtcc tcggacccat tccaacaatc tcgtaaaaca tggtggatta     180

ctatgaagtt ctaggcgtgc agagacatgc ctcacccgag gatattaaaa aggcatatcg     240

gaaactggca ctgaa                                                      255


<210>  42
<211>  255
<212>  DNA
<213>  Homo Sapiens


<220>
<221>  misc_feature
<222>  (108)..(108)
<223>  n is a, c, g, or t

<400>  42
gaatcgtcga cccacgcgtc cgcggacgcg tgggccacca tctctagttc ttcttccact      60

aaaggaataa agaggaaagc tacagaaatt agcactgcag tggttcanag gtcagctacc     120

attggcagtt ctccagttct ctatagccag tcagctatag ctacaggtca ccaggcagca     180

gggattggaa accaggcaac aggaattgga catcagacaa taccagttag ccttccagca     240

gcaggaatgg gtcat                                                      255


<210>  43
<211>  255
<212>  DNA
<213>  Homo Sapiens

<400>  43
gaatcgtcga cccacgcgtc cgcgcgggag aggagcccca ctcccccagc gccgcagcca      60

ccgcagccac cgcagcccgt gcgccccgcg ccctcgagcg ccatggccaa ggaaggcgtg     120

gagaaggcgg aggagacgga gcaaatgatc gagaaggagg caggcaagga gccggcggag     180

ggcggcggcg gcgacggctc gcaccgcctc ggggacgccc aggagatgcg cgcggtggtg     240

ctggctggct tcggg                                                      255


<210>  44
<211>  255
<212>  DNA
<213>  Homo Sapiens

<400>  44
gaatcgtcga cccacgcgtc cgggggtctc agagcagacc acctgccagg aagtggtcat      60

cgcactagcc caagcaatag gccagactgg ccgctttgtg cttgtgcagc ggcttcggga 120

gaaggagcgg cagttgctgc cacaagagtg tccagtgggc gcccaggcca cctgcggaca 180

gtttgccagc gatgtccagt ttgtcctgag gcgcacaggg cccagcctag ctgggaggcc 240

ctcctcagac agctg 255

<210> 45
<211> 255
<212> DNA
<213> Homo Sapiens

<400> 45
gaatcgtcga cccacgcgtc cgaaaggcga ccctcgaaag ataagattga agatgagctg 60

gagatgacca tggtttgcca tcggcccgag ggactggagc agctcgaggc ccagaccaac 120

ttcaccaaga gggagctgca ggtcctttat cgaggcttca aaaatgagtg ccccagtggt 180

gtggtcaacg aagacacatt caagcagatc tatgctcagt ttttccctca tggagatgcc 240

agcacgtatg cccat 255

<210> 46
<211> 255
<212> DNA
<213> Homo Sapiens

<400> 46
gaatcgtcga cccacgcgtc cgggccgctg tacatgagca ccaagaacac catactgaaa 60

gcctacgatg ggcgtttcaa ggacatcttc caggagatct ttgacaagca ctataagacc 120

gacttcgaca agaataagat ctggtatgag caccggctca ttgatgacat ggtggctcag 180

gtcctcaagt cttcgggtgg ctttgtgtgg gcctgcaaga actatgacgg agatgtgcag 240

tcagacatcc tggcc 255

<210> 47
<211> 255
<212> DNA
<213> Homo Sapiens

<400> 47
gaatcgtcga cccacgcgtc cgcttttagc accagtgtgg gaaatgagga cgccaggaca 60

gcctggcccg aattacaaca gagccatgct gttaatcagc tcaaagattt gttgcgccaa 120

caagcagata aggaaagtga agtatctccg tcaagaagaa gaaaaatgtc ccccttgagg 180

tcattagaac atgaggaaac caatatgcct actatgcacg accttgttca tactattaat 240

gaccagtctc aatat 255

<210> 48
<211> 255
<212> DNA

<213>  Homo Sapiens

<220>
<221>  misc_feature
<222>  (1)..(1)
<223>  n is a, c, g, or t

<220>
<221>  misc_feature
<222>  (25)..(25)
<223>  n is a, c, g, or t

<220>
<221>  misc_feature
<222>  (34)..(34)
<223>  n is a, c, g, or t

<220>
<221>  misc_feature
<222>  (47)..(47)
<223>  n is a, c, g, or t

<220>
<221>  misc_feature
<222>  (69)..(69)
<223>  n is a, c, g, or t

<220>
<221>  misc_feature
<222>  (116)..(116)
<223>  n is a, c, g, or t

<220>
<221>  misc_feature
<222>  (141)..(141)
<223>  n is a, c, g, or t

<220>
<221>  misc_feature
<222>  (189)..(189)
<223>  n is a, c, g, or t

<220>
<221>  misc_feature
<222>  (212)..(212)
<223>  n is a, c, g, or t

<220>
<221>  misc_feature
<222>  (238)..(238)
<223>  n is a, c, g, or t

<220>
<221>  misc_feature
<222>  (245)..(245)
<223>  n is a, c, g, or t

<220>
<221>  misc_feature
<222>  (249)..(249)
<223>  n is a, c, g, or t

<220>
<221>  misc_feature
<222>  (252)..(252)
<223>  n is a, c, g, or t

<220>
<221>  misc_feature
<222>  (255)..(255)
<223>  n is a, c, g, or t

<400>  48
naatcgtcga cccacgcgtc cggantacgg gggnaagaac tgctcanaag ccggccgagt      60

acggctacnt gagcctgggg cgcctggggg ccgccaaggg cgcgctgcac cccggntgct     120

tcgcgcccct gcacccaccg nccccgccgc cgccgccgcc cgccgagctc aaggcggagc     180

cgggcttcna gcccgcggac tgcaagcgga angaggaggc cggggcgccg ggcggcgncg     240

caagnatgnc gncgn                                                      255


<210>  49
<211>  255
<212>  DNA
<213>  Homo Sapiens

<400>  49
gaatcgtcga cccacgcgtc cgcccacgcg tccgggacgc gtgcggggca ggtcacgctt      60

gggcctggct gcctcaggag ccaccctgca cggcgccggg cgaggctgtg cgcgccctcc     120

cggaccctcc actttggggc tttgcggtgt agcggcgcgc cccactccct tacgtgttca     180

gtcccgaatc acgtacgggc cgctgcagct ccaggacggc ggacgccagc gctctcggat     240

ccccaggccc ctccg                                                      255


<210>  50
<211>  255
<212>  DNA
<213>  Homo Sapiens

<400>  50
gaatcgtcga cccacgcgtc cgcagggtct ccggtagggg aaggcactgg atcgcctccc      60

aagtggcaga ttggtgagca ggaatttgaa gccctcatgc ggatgctcga taatctgggc     120

tacagaactg gctaccctta tcgataccct gctctgagag agaagtctaa aaaatacagc     180

gatgtggagg ttcctgctag tgtcacaggt tactcctttg ctagtgacgg tgattcgggc     240

acttgctccc cactc                                                      255


<210>  51
<211>  255
<212>  DNA
<213>  Homo Sapiens

<400>  51
gaatcgtcga cccacgcgtc cgctctctcg tccggtgtct gcgtgccaag ctgtattacc      60

aactgcttgt ggactaaaga ggatggacat agaacttcca cctctgctgt ccctaacctg 120

tttgttccat tgaacactaa cccaaaagag gtccaggaga tgaggaacaa gatccgagag 180

cagaatttac aggacattaa gacggctggc cctcagtccc aggttttgtg tggtgtagtg 240

atggacagga gcctc 255

<210> 52
<211> 255
<212> DNA
<213> Homo Sapiens

<400> 52
gaatcgtcga cccacgcgtc cgggcggaac aggctaccaa gtccgtgctg tttgtgtgtc 60

tgggtaacat ttgtcgatca cccattgcag aagcagtttt caggaaactt gtaaccgatc 120

aaaacatctc agagaattgg agggtagaca gcgcggcaac ttccgggtat gagataggga 180

acccccctga ctaccgaggg cagagctgca tgaagaggca cggcattccc atgagccacg 240

ttgcccggca gatta 255

<210> 53
<211> 255
<212> DNA
<213> Homo Sapiens

<400> 53
gaatcgtcga cccacgcgtc cgctgatttc cacctagcaa tgcccacgga gcaggcagag 60

ggcttctaca acagcttcct ggagcagctg cgtaaaacat acaggccgga gcttatcaaa 120

gatggcaagt ttggggccta catgcaggtg cacattcaga atgatgggcc tgtgaccata 180

gagctggaat cgccagctcc cggcactgct acctctgacc caaagcagct gtcaaagctc 240

gaaaaacagc agcag 255

<210> 54
<211> 255
<212> DNA
<213> Homo Sapiens

<400> 54
gaatcgtcga cccacgcgtc cgaggccact atggaggaaa acacctatcc ctccttcctt 60

aagtctgata tttatttgga atatacgagg acaggctcgg agagccccaa agtctgtagt 120

gaccagagct ctgggtcagg gacagggaag ggcatatctg gatacctgcc gaccttaaat 180

gaagatgagg aatggaagtg tgaccaggac atggacgagg acgatggcag agacgctgct 240

ccccccggaa gactc 255

<210> 55
<211> 255

<212>  DNA
<213>  Homo Sapiens

<400>  55
gaatcgtcga cccacgcgtc cgagaagact cgccgaaagt tcctggacct gggggtcacc      60

ctgcgccgag catccacggg caagagccgg aaggagaaag gcagcaaccg actgtccatg     120

ggcagcaggg agtcagtgga ggggtccggc aggtcagggg gctccccgtt cctgcctttt     180

tcctggttca cggacagcgg caagggctca gcatcctcgg gtagcaccac ctcccccacc     240

tgctccccta aacac                                                      255


<210>  56
<211>  255
<212>  DNA
<213>  Homo Sapiens


<220>
<221>  misc_feature
<222>  (24)..(24)
<223>  n is a, c, g, or t

<220>
<221>  misc_feature
<222>  (152)..(152)
<223>  n is a, c, g, or t

<400>  56
gaatcgtcga cccacgcgtc cggncggtcc ccgggcccac tggcggctcc tcgggtcgct      60

tcctcagccc cgagcagcac gcgtgcagct ggcagctcct gctgcccgcc ccggaggccg     120

cagcgggcag cgagctggcg ctgcgctgcc anagcccgga cggggcgcgc caccagtgcg     180

cctaccgcgg gcatccggag cgctgcgcag cctacgccgc tcgccgcgcg cacttctgga     240

agcaggtgct gggag                                                      255


<210>  57
<211>  255
<212>  DNA
<213>  Homo Sapiens

<400>  57
gaatcgtcga cccacgcgtc cgggccgcgg gagacgtggt gccgctgcgg gctcgctctg      60

ccgtgcgcta ggcttggtgg gaaggcctgt tctcgagtcc gcgcttttcg tcaccgccat     120

gtcgggaggt ggtgtgattc gtggccccgc agggaacaac gattgccgca tctacgtggg     180

taacttacct ccagacatcc gaaccaagga cattgaggac gtgttctaca aatacggcgc     240

tatccgcgac atcga                                                      255


<210>  58
<211>  255
<212>  DNA

<213>  Homo Sapiens

<400>  58
gaatcgtcga cccacgcgtc cgcccacgcg tccgcactgg tgtgtcctcc ccttccgagt      60

gctgctggta ctcacctcac atcgaggaat acagatgtac gagtccaatg gctacaccat     120

ggtctactgg catgcactgg actctggaga tgcctcccca gtacaggctg tgtttgcccg     180

gggaattgct gccagtggcc acttcatctg tgtgggaacg tggtcaggcc gggtgctggt     240

gtttgacatc ccagc                                                      255


<210>  59
<211>  255
<212>  DNA
<213>  Homo Sapiens

<400>  59
gaatcgtcga cccacgcgtc cgcccacgcg tccgcggcgg cgggagcggt catggaggcg      60

ggcgccggag ccggcgcggg agccgcgggc tggagctgcc cgggcccagg acccacagtg     120

accactctag gctcctatga ggcttccgag ggctgtgaga ggaagaaggg ccaacgctgg     180

gggtccctgg aacgacgggg gatgcaagct atggaggggg aggtgttact cccagctctc     240

tatgaggagg aagag                                                      255


<210>  60
<211>  255
<212>  DNA
<213>  Homo Sapiens

<400>  60
gaatcgtcga cccacgcgtc cgcgcaagca ggaggcgggg gagtcggagg aggtggcggc      60

gctggagctc ctcccgggga ccagcgaccc ggggagcgag cacgtcgctc cgcaccgctc     120

ttcctccagc cgctgagccg tcccttctcg ccatgtccca gagcaggcac cgcgccgagg     180

ccccgccgct ggagcgcgag gacagtggga ccttcagttt ggggaagatg ataacagcta     240

agccagggaa aacac                                                      255


<210>  61
<211>  255
<212>  DNA
<213>  Homo Sapiens

<400>  61
gaatcgtcga cccacgcgtc cgagaaacct gcagccgcag cagctcctgg cactgcggag      60

aagttgagcc ccaaggcggc cacgcttgcc gagcgcagcg ccggcctggc cttcagcttg     120

taccaggcca tggccaagga ccaggcagtg gagaacatcc tggtgtcacc cgtggtggtg     180

gcctcgtcgc tggggctcgt gtcgctgggc ggcaaggcga ccacggcgtc gcaggccaag     240

gcagtgctga gcgcc                                                      255

<210>  62
<211>  255
<212>  DNA
<213>  Homo Sapiens

<400>  62
gaatcgtcga cccacgcgtc cgccccacgc cgcggcgcgc ccgggctccc tgctgatccc      60

agaacaatca accatgacga ccgaatctgg atcagactcg gaatccaagc cggaccagga     120

ggccgagccc caggaggcgg cgggggcgca ggggcgcgcg ggggcgcccg tgccggagcc     180

gcccaaggag gagcagcagc aggccctgga gcagttcgcc gccgctgcag cgcacagcac     240

cccggtgcgg aggga                                                      255


<210>  63
<211>  255
<212>  DNA
<213>  Homo Sapiens

<400>  63
gaatcgtcga cccacgcgtc cgtgagtctc aagatggaca accgggatgt tgcaggaaag      60

gctaaccggt ggtttggggt tgctccccct aaatctggaa aaatgaacat gaacatcctt     120

caccaggaag agctcatcgc tcagaagaaa cgggaaattg aagccaaaat ggaacagaaa     180

gccaagcaga atcaggtggc cagccctcag cccccacatc ctggcgaaat cacaaatgca     240

cacaactctt cctgc                                                      255


<210>  64
<211>  255
<212>  DNA
<213>  Homo Sapiens

<400>  64
agaatcgtcg acccacgcgt ccgcccacgc gtccgccatg ccagatgacc ttctgaccac      60

gttggatgac acttgtgatc tctttgcccc cctagtccag gagactaata agcagccccc     120

ccagacggtg aacccaattg gccatctccc agaaagttta acaaaacccg accaggttgc     180

ccccttcacc ttcttacaac ctcttcccct ttccccaggg gtcctgtcct ccactccaga     240

tcattccttt agttt                                                      255


<210>  65
<211>  255
<212>  DNA
<213>  Homo Sapiens

<400>  65
gaatcgtcga cccacgcgtt ccgattgtga tggaccccaa gaaggacgtc ctcatcgagt      60

tctacgcgcc atggtgcggg cactgcaagc agctagagcc cgtgtacaac agcctggcca     120

agaagtacaa gggccaaaag ggcctggtca tcgccaagat ggacgccact gccaacgacg 180

tccccagcga ccgctataag gtggagggct tccccaccat ctacttcgcc cccagtgggg 240

acaaaaagaa cccag 255

<210> 66
<211> 255
<212> DNA
<213> Homo Sapiens

<400> 66
gaatcgtcga cccacgcgtc cggccccttg gagagagagg ctgcctcagc cagtggcaca 60

ggagactcca aggagctact gacattccta agagtggagg aggaggagga gccttgctgg 120

gccagggaaa caaagtttac attgtcctgt agctttaaaa ccacagctgg gcagggtgag 180

aagctagatg cccctgcagt ttggccctgg agccagggca gaggaatgta gggcctgcat 240

ggagaagggt tctgc 255

<210> 67
<211> 255
<212> DNA
<213> Homo Sapiens

<400> 67
gaatcgtcga cccacgcgtc cggccagctc caccagcacg tgcagcagct acacctgggc 60

acccggctgc ctgtggtggt cgtggccaac aaagctgacc tgttgcacat caaacaggtt 120

gaccctcagc ttggactgca gctagccagc atgctaggct gctcattcta tgaagtgtct 180

gtcagtgaaa attataatga tgtctacagc gccttccacg tcctctgtaa agaggtcagt 240

cacaaacagc agcct 255

<210> 68
<211> 255
<212> DNA
<213> Homo Sapiens

<400> 68
gaatcgtcga cccacgcgtc cggcctccgc cttcggaggc tgacgcgccc gggcgccgtt 60

ccaggcctgt gcagggcgga tcggcagccg cctggcggcg atccagggcg gtgcggggcc 120

tgggcgggag ccgggaggcg cggccggcat ggaggcgctg ctgctgggcg cggggttgct 180

gctgggcgct tacgtgcttg tctactacaa cctggtgaag gccccgccgt gcggcggcat 240

gggcaacctg cgggg 255

<210> 69
<211> 255
<212> DNA
<213> Homo Sapiens

<400>  69
gaatcgtcga cccacgcgtc cgcgggcggc ggcggagcgg gcggcggagc ggcggcagca      60

gcggtgagcg cgagccccgg agccccgggc ggccggcctc ccgcgcccgc gcagccccgc     120

gtctgcgtcc ggccggagac gccgggcccc gcgccgcgcc gcgccatgtc ggtgaacatg     180

gacgagctgc ggcaccaggt catgatcaac cagttcgtgc tggccgcggg ctgcgcggcc     240

gaccaggcga agcag                                                      255


<210>  70
<211>  255
<212>  DNA
<213>  Homo Sapiens

<400>  70
gaatcgtcga cccacgcgtc cggagtgtac aatccgtggc cggagcctga tccagattag      60

catccaggag gacccctgga acctccccaa ctccatcaag accctggtgg acaacattca     120

gagatatgtg gaagatggga agaaccagct gctcctggcc ttgctgaagt gcacagacac     180

ggagctgcag ctgcgcagag acgcgatctt ctgccaggcc ctggtggccg ccgtgtgcac     240

cttctccgag cagct                                                      255


<210>  71
<211>  255
<212>  DNA
<213>  Homo Sapiens

<400>  71
gaatcgtcga cccacgcgtc cgaccaggca tgcagacaag tgcctgcagt gattgcagcg      60

cctgcgcagc ggcccttgtt ggaagtccag gagtccgcgc taccccgcaa agtccctggc     120

cgctgcccgc acaccgaacc tctcggggct gagtcgggct gctttctcct cgcgtcccgg     180

ggcgccctcg ggaactcggg agcgcgcggc gagctgaaca atggcctggg gacacctggc     240

aggtcgcagg cccca                                                      255


<210>  72
<211>  255
<212>  DNA
<213>  Homo Sapiens


<220>
<221>  misc_feature
<222>  (1)..(1)
<223>  n is a, c, g, or t

<220>
<221>  misc_feature
<222>  (26)..(26)
<223>  n is a, c, g, or t

<220>

<221>  misc_feature
<222>  (37)..(37)
<223>  n is a, c, g, or t

<220>
<221>  misc_feature
<222>  (122)..(122)
<223>  n is a, c, g, or t

<220>
<221>  misc_feature
<222>  (172)..(172)
<223>  n is a, c, g, or t

<220>
<221>  misc_feature
<222>  (224)..(224)
<223>  n is a, c, g, or t

<220>
<221>  misc_feature
<222>  (226)..(226)
<223>  n is a, c, g, or t

<220>
<221>  misc_feature
<222>  (242)..(242)
<223>  n is a, c, g, or t

<400>  72
naatcgtcga cccacgcgtt ccgacnaaaa cgaaaangaa aatgggtccc aggccctccc      60

cgccagcctc cgtagacggg aaggatcagg aagcggggac tccgggatgt cagcggcgcc     120

gnggcgcggg ctccgggctt ccctgccctg caatgtggaa gctgctctct angaggggcc     180

cgaggctggg cccacggatg cgggcgcgcc gggaggatat ttancnttga agagcggaag     240

gnggcggaga ctcgg                                                      255


<210>  73
<211>  255
<212>  DNA
<213>  Homo Sapiens

<400>  73
gaatcgtcga cccacgcgtc cgcgggcgca cgcgcgctcc tggagtttcc caaccttccg      60

ggtgcgggcg gggcatgtta cgtcacgtcg tttaatccgg aaacggcggc ggcggcgaca     120

ggaccgaggg gccttagttg gtgggcaagt cggggatccc agaaagagaa gcgtgacccg     180

gaagcggaaa cgggtgtccg tcccagctcc ggcctgccag tgagcttcta ccatcatgga     240

cctattgttc gggcg                                                      255


<210>  74
<211>  255
<212>  DNA
<213>  Homo Sapiens

<220>
<221>  misc_feature
<222>  (162)..(162)
<223>  n is a, c, g, or t

<400>  74
gaatcgtcga cccacgcgtc cgggtttggt tgtgagatcg agaataacag aagcagcgga      60

gcattctgga aatattacta tgatggaaag gactacattg aattcaacaa agaaatccca     120

gcctgggtcc ccttcgaccc agcagcccag ataaccaagc anaagtggga ggcagaacca     180

gtctacgtgc agcgggccaa ggcttacctg gaggaggagt gccctgcgac tctgcggaaa     240

tacctgaaat acagc                                                      255


<210>  75
<211>  255
<212>  DNA
<213>  Homo Sapiens

<400>  75
gaatcgtcga cccacgcgtc cgtgaaggcc gaggccaaga tggcggtgct gtcaggtgag      60

cgcggcaccg gcggcgggtg tggggccgcg cgggtctggg gccgtgggag cctcgggtgt     120

cgtggtggcg tcgggggtcg gtgccggcgt cgtggccgcg gtcctctccg ggcttctccg     180

agccggccgc tcctcgggct ccccgcccgg cttgcgatga acggtcgccg ttattgcgtc     240

cagagtacag tcggg                                                      255


<210>  76
<211>  255
<212>  DNA
<213>  Homo Sapiens


<220>
<221>  misc_feature
<222>  (172)..(172)
<223>  n is a, c, g, or t

<220>
<221>  misc_feature
<222>  (227)..(227)
<223>  n is a, c, g, or t

<220>
<221>  misc_feature
<222>  (232)..(232)
<223>  n is a, c, g, or t

<400>  76
   gaatcgtcga cccacgcgtc cgccgagtcc gcggcggcgt ccaggtcggc cagcaaccgc      60
   agccgagccc gagcgggtgg cggcgccatg gcgtgcgcgg ggctgctcac cgtgtgcctg     120
   ctccggccgc ccgcgcccca gccccagccc cagaccccgc ggcaccccca gntcgcgccc     180
   gacccggggc ccgccggaca cacgctcttc caggacgttt tccgcanagc anacaagaat 240
   ggagtacgag agggc 255

<210> 77
<211> 255
<212> DNA
<213> Homo Sapiens

<400> 77
gaatcgtcga cccacgcgtc cgtccacatg gagtacagct gcggcacagc ggccatccgg 60

ggcaccaagg agctggggga gggccagcac ttctgggaga tcaagatgac ctctcccgtc 120

tacggcaccg acatgatggt gggcatcggg acgtcggatg tggacctgga caaataccgc 180

cacacgttct gcagcctgct gggcagggat gaggacagct ggggcctctc ctacacgggc 240

ctcctccacc acaag 255

<210> 78
<211> 255
<212> DNA
<213> Homo Sapiens

<400> 78
gaatcgtcga cccacgcgtc cgccaagatg ccgcccaaag gaaaaagtgg ttctggaaaa 60

gcggggaaag ggggagcagc ctctgggagt gacagtgctg acaagaaggc tcaaggtccc 120

aaaggtggtg gcaatgcagt aaaggtcaga cacattctat gtgaaaaaca tggcaaaatc 180

atggaagcca tggaaaagtt aaagtctggg atgagattca atgaagtggc cgcacagtat 240

agtgaagata aagcc 255

<210> 79
<211> 255
<212> DNA
<213> Homo Sapiens

<220>
<221> misc_feature
<222> (140)..(140)
<223> n is a, c, g, or t

<220>
<221> misc_feature
<222> (146)..(146)
<223> n is a, c, g, or t

<220>
<221> misc_feature
<222> (233)..(233)
<223> n is a, c, g, or t

<400> 79
tagaatcgtc gacccacgcg ctccgcgagt cgatcggccg ccgcgctcga gcctccgccc 60

gcaccgagcc gcgggacccg ggccgtaccg gggaggggcc gctccgggcc gcaacgcgag 120

ggcagcgagg ggcggcgggn acctgncacc gggcggggcc ggcggcagcg accatgatcg 180

ctttgttcaa caagctgctg gactggttca aggccctatt ctggaaggag ganatggagc 240

tcacgctggt cgggc 255

<210> 80
<211> 255
<212> DNA
<213> Homo Sapiens

<400> 80
gaatcgtcga cccacgcgtc cggactggcg ccacggcagc ggtgctgcag agcgacacca 60

tggaccatta ccgcaccttc cacatgctcg agcggctgct gcatgcgccg cccaagctac 120

tgcaccagct catcttccag attccgccct cccggcaggc actactcatc gagaggtact 180

atgcctttga tgaggccttt gttcgggagg tgctgggcaa gaagctgtcc aaaggcacca 240

agaaagacct ggatg 255

<210> 81
<211> 255
<212> DNA
<213> Homo Sapiens

<400> 81
gaatcgtcga cccacgcgtc cggaaaagcc atcgagctca acccagccaa cgccgtctat 60

ttctgcaaca gagccgcagc ctacagcaaa ctcggcaact acgcaggcgc ggtgcaggac 120

tgtgagcggg ccatctgcat tgacccggcc tacagcaagg cctacggcag gatgggcctg 180

gcgctctcca gcctcaacaa gcacgtggag gccgtggctt actacaagaa ggcgctggag 240

ctggaccccg acaac 255

<210> 82
<211> 255
<212> DNA
<213> Homo Sapiens

<400> 82
gaatcgtcga cccacgcgtc cgcagcagag ctggaggctg aggagctcga gaaattgcaa 60

caatacaaat tcaaagcacg tgaacttgat cccagaatac ttgaaggtgg gcccatcttg 120

cccaagaaac cacctgtgaa accacccacc gagcctattg gctttgattt ggaaattgag 180

aaaagaatcc aggagcgaga atcaaagaag aaaacagagg atgaacactt tgaatttcat 240

tccagacctt gccct 255

<210> 83
<211> 255
<212> DNA

<213>  Homo Sapiens

<400>  83
gaatcgtcga cccacgcgtc cgctggaggc gcgggccggg cggtgcgcac tgcgggcgca      60

tccctgcccc ggcgccgtcc gtgcccgcgg gacctgacag ccgggtcaga gggcgaagct     120

gtgctcaggc ccgggctgga cgcagagcca gagctgtccc cagaggagca gagggtcctg     180

gaaaggaagc tgaaaaagga acggaagaaa gaggagaggc agcgtctgcg ggaggcaggc     240

cttgtggccc agcac                                                      255


<210>  84
<211>  255
<212>  DNA
<213>  Homo Sapiens

<400>  84
gaatcgtcga cccacgcgtc cgctcagtac cgtgtgactg tgccgctgat gggggctgtg      60

tccgacctgt gcgaggctct ctccaggctg tctggcattg ctgcagaaaa tatggtggtc     120

gcagatgtgt ataatcaccg attccacaaa attttccaaa tggatgaagg tttaaaccac     180

atcatgcctc gggatgacat tttcgtgtac gaggtctgca gcacttccgt ggatggctcg     240

gaatgtgtca cgctt                                                      255


<210>  85
<211>  246
<212>  DNA
<213>  Homo Sapiens


<220>
<221>  misc_feature
<222>  (1)..(1)
<223>  n is a, c, g, or t

<220>
<221>  misc_feature
<222>  (9)..(9)
<223>  n is a, c, g, or t

<220>
<221>  misc_feature
<222>  (14)..(14)
<223>  n is a, c, g, or t

<220>
<221>  misc_feature
<222>  (16)..(16)
<223>  n is a, c, g, or t

<220>
<221>  misc_feature
<222>  (74)..(74)
<223>  n is a, c, g, or t

<220>

<221>  misc_feature
<222>  (80)..(81)
<223>  n is a, c, g, or t

<220>
<221>  misc_feature
<222>  (88)..(88)
<223>  n is a, c, g, or t

<220>
<221>  misc_feature
<222>  (99)..(99)
<223>  n is a, c, g, or t

<220>
<221>  misc_feature
<222>  (103)..(103)
<223>  n is a, c, g, or t

<220>
<221>  misc_feature
<222>  (121)..(122)
<223>  n is a, c, g, or t

<220>
<221>  misc_feature
<222>  (137)..(137)
<223>  n is a, c, g, or t

<220>
<221>  misc_feature
<222>  (139)..(139)
<223>  n is a, c, g, or t

<220>
<221>  misc_feature
<222>  (145)..(146)
<223>  n is a, c, g, or t

<220>
<221>  misc_feature
<222>  (156)..(156)
<223>  n is a, c, g, or t

<220>
<221>  misc_feature
<222>  (164)..(164)
<223>  n is a, c, g, or t

<220>
<221>  misc_feature
<222>  (166)..(166)
<223>  n is a, c, g, or t

<220>
<221>  misc_feature
<222>  (169)..(169)
<223>  n is a, c, g, or t

<220>
<221>  misc_feature
<222>  (173)..(174)
<223>  n is a, c, g, or t

<220>
<221>  misc_feature
<222>  (177)..(177)
<223>  n is a, c, g, or t

<220>
<221>  misc_feature
<222>  (188)..(188)
<223>  n is a, c, g, or t

<220>
<221>  misc_feature
<222>  (192)..(192)
<223>  n is a, c, g, or t

<220>
<221>  misc_feature
<222>  (205)..(206)
<223>  n is a, c, g, or t

<220>
<221>  misc_feature
<222>  (212)..(212)
<223>  n is a, c, g, or t

<220>
<221>  misc_feature
<222>  (219)..(219)
<223>  n is a, c, g, or t

<220>
<221>  misc_feature
<222>  (222)..(222)
<223>  n is a, c, g, or t

<220>
<221>  misc_feature
<222>  (227)..(227)
<223>  n is a, c, g, or t

<220>
<221>  misc_feature
<222>  (236)..(236)
<223>  n is a, c, g, or t

<220>
<221>  misc_feature
<222>  (241)..(241)
<223>  n is a, c, g, or t

<220>
<221>  misc_feature
<222>  (243)..(243)
<223>  n is a, c, g, or t

<220>
<221>  misc_feature
<222>  (245)..(245)
<223>  n is a, c, g, or t

<400>  85
naatcgtcna cccncncgct ccgccctggc ggcggcggcg gcgcctgaca ctcggcgcct      60

cctgccgtgc tcangggcgn natgtccnag gctggaggng ccngcccggg aggctgatcc 120

nncaagatcc gggacangnt cctgnncccg gggcgnctga cccngnatnc tgnngcnctt 180

ccgcccgngc cnccgtatgg ctccnnctgc gntattgcng cnggggngtc ggtctnctgc 240

nantnc 246

<210> 86
<211> 255
<212> DNA
<213> Homo Sapiens

<400> 86
gaatcgtcga cccacgcgtc cggccatcgg cgtgctgacc agcggcgggg atgctcaagg 60

tatgaacgct gccgtccgtg ccgtggtgcg catgggtatc tacgtggggg ccaaggtgta 120

cttcatctac gagggctacc agggcatggt ggacggaggc tcaaacatcg cagaggccga 180

ctgggagagt gtctccagca tcctgcaagt gggcgggacg atcattggca gtgcgcggtg 240

ccaggccttc cgcac 255

<210> 87
<211> 255
<212> DNA
<213> Homo Sapiens

<400> 87
gaatcgtcga cccacgcgtc cgaaaaaaga gaacgagaaa gacaagaact gagaatttta 60

gtgggaacaa atttggtgcg cctcagtcag ttggaaggtg taaatgtgga acgttacaaa 120

cagattgttt tgactggcat attggagcaa gttgtaaact gtagggatgc tttggctcaa 180

gaatatctca tggagtgtat tattcaggtt ttccctgatg aatttcacct ccagactttg 240

aatccttttc ttcgg 255

<210> 88
<211> 255
<212> DNA
<213> Homo Sapiens

<400> 88
gaatcgtcga cccacgcgtc cgctccccac agccagagga ggccacagac ccctcagggg 60

agttccgcgc tggggtctgg gctgtgctcc ctcactaaag ggaaggaaag gaagctgggc 120

gtcctccggg ccccccaaca cacgtcccat ttagccctgc acagcggtct ccttccccta 180

agccagcact gctgctccct ggagcccggg aaggaggctg cctggctgga ggcccgagcc 240

gatggcgcct gtgct 255

<210> 89
<211> 255

<212>  DNA
<213>  Homo Sapiens

<400>  89
gaatcgtcga cccacgcgtc cggctgccta tttccaccaa gcagccaagc tcaagggaat      60

cggggagtac gtgaacatcc gcacagggat gccctgccac ttgcacccca ccagctccct     120

ttttggaatg ggctacaccc cagattacat agtgtatcac gagttggtca tgaccaccaa     180

ggagtatatg cagtgtgtga ccgctgtgga cggggagtgg ctggcggagc tgggccccat     240

gttctatagc gtgaa                                                      255


<210>  90
<211>  255
<212>  DNA
<213>  Homo Sapiens

<400>  90
gaatcgtcga cccacgcgtc cgacatcctg cagcagacac tgatggacga ggggctgcgg      60

ctcgcccgcc tcgtctccca cgaccgcgtg ggccgcctca gcccctgtgt gcctgcgaag     120

ccacctctcg cagagttcga ggaagggctg ctggacagat gtcctgcccc aggaccccat     180

cccgcgctgg tggagggtcg caggagcagc gtgaaagtgg aggctgaggc cagccggcag     240

tgagggttgg actgg                                                      255


<210>  91
<211>  255
<212>  DNA
<213>  Homo Sapiens

<400>  91
gaatcgtcga cccacgcgtc cgattgcccg ctccaccctc cacatgccct ttctaactcc      60

tcagttgacc tagggtcccc aagccttgcc agtcaccccc ggggcctgct gcagaaccgt     120

ggcctcaaca atgacagtcc tgagcggagg cgcaggcctg gccatggcag cctgaccaac     180

atcagccggc acgactccct caagaagatc gacagccctc ccattagaag gtccacgtca     240

tcagggcaat acacg                                                      255


<210>  92
<211>  255
<212>  DNA
<213>  Homo Sapiens


<220>
<221>  misc_feature
<222>  (143)..(143)
<223>  n is a, c, g, or t

<220>
<221>  misc_feature
<222>  (158)..(158)

<223>  n is a, c, g, or t

<220>
<221>  misc_feature
<222>  (161)..(161)
<223>  n is a, c, g, or t

<400>  92
gaatcgtcga cccacgcgtc cggccgctgc caccgcaccc cgccatggag cggccgtcgc      60

tgcgcgccct gctcctcggc gccgctgggc tgctgctcct gctcctgccc ctctcctctt     120

cctcctcttc ggacacctgc ggnccctgcg agccggcntc ntgcccgccc ctgcccccgc     180

tgggctgcct gctgggcgag acccgcgacg cgtgcggttg ctgccctatg tgcgcccgcg     240

gcgagggcga gccgt                                                      255


<210>  93
<211>  255
<212>  DNA
<213>  Homo Sapiens

<400>  93
gaatcgtcga cccacgcgtc cgcggacgcg tgggtggggc cccgggcggc gttgaccatg      60

acccagcagg gcgcggcgct gcagaactac aacaacgagc tggtcaagtg catagaggag     120

ctgtgccaga agcgggagga gctgtgccgg cagatccagg aggaggagga cgagaagcag     180

cggctgcaga atgaggtgag gcagctgaca gagaagctgg cccgcgtcaa cgagaacctg     240

gcacgcaaga ttgcc                                                      255


<210>  94
<211>  255
<212>  DNA
<213>  Homo Sapiens

<400>  94
gaatcgtcga cccacgcgtc cggcctgcag agcaacatgc ccaagtttta ttgtgactac      60

tgcgatacat acctcaccca tgactctcca tctgtgagaa agacacactg cagtggaagg     120

aaacacaaag agaatgtgaa agactattat cagaaatgga tggaagagca ggctcagagc     180

ctgattgaca aaacaacggc tgcatttcaa caaggaaaga tacctcctac tccattctct     240

gctcctcctc ctgca                                                      255


<210>  95
<211>  255
<212>  DNA
<213>  Homo Sapiens

<400>  95
gaatcgtcga cccacgcgtc cgcccacgcg tccgctagtc caagtgtccg gcaaacttgg      60

atccatgaaa tcaaccaaat tttagaaaac cagcgcaatt ttttaaatgc cttgacatcg     120

ccaatcgagt accagaggaa ccacagcggg ggcggcggcg gcggcggcag cgggggcagc 180

ggcgggggtg ggggcagcgg cggcggcggg gcccccagtg gcggcagcgg ccacagtggc 240

ggccccagca gctgc 255

<210> 96
<211> 255
<212> DNA
<213> Homo Sapiens

<400> 96
gaatcgtcga cccacgcgtc cgatgaaatt gaagaaatgg gtggaatggc caaagctgta 60

gctgagggaa tacctaaact tcgaattgaa gaatgtgctg cccgaagaca agctagaata 120

gattctggtt ctgaagtaat tgttggagta aataagtacc agttggaaaa agaagacgct 180

gtagaagttc tggcaattga taatacttca gtgcgaaaca ggcagattga aaaacttaag 240

aagatcaaat ccagc 255

<210> 97
<211> 255
<212> DNA
<213> Homo Sapiens

<400> 97
gaatcgtcga cccacgcgtc cgcggaccct cgacgctgca tgaggcacca ctatgtggat 60

tctatcagtc acccattgta caagtgtagc tcaaagatgg tgctcctggc caggtgcgag 120

gggcactgca gccaggcgtc acgctccgag cctttggtgt cgttcagcac tgtcctcaag 180

caacccttcc gttcctcctg tcactgctgc cggccccaga cttccaagct gaaggcactg 240

cggctgcgat gctca 255

<210> 98
<211> 255
<212> DNA
<213> Homo Sapiens

<220>
<221> misc_feature
<222> (1)..(1)
<223> n is a, c, g, or t

<400> 98
naatcgtcga cccacgcggt ccgggacgcc gacggcggcg ggcgcttccg cctcgcgggc 60

ctcggcccgg tgcgagcggc tccgcgatgt ggctgagccc ggaggaggtg ctggtggcca 120

atgcgctgtg ggtgacggag cgggccaacc ccttcttcgt gctgcagcga cgccggggcc 180

acggcagggg cggcggcctt acgggtcttc tcgtgggcac cctggacgtg gtgctggact 240

ccagtgcccg cgtgg 255

<210> 99
<211> 255
<212> DNA
<213> Homo Sapiens


<220>
<221> misc_feature
<222> (34)..(34)
<223> n is a, c, g, or t

<220>
<221> misc_feature
<222> (42)..(42)
<223> n is a, c, g, or t

<400> 99
gaatcgtcga cccacgcgtt ccgcccacgc gtcnggtacg cntccccacg aggggctggt 60

tcctgaaagg atgtacagag gatccccaac cgcctgcgaa acccaagccg ccgcgtagga 120

gcgtgcgttc gggccctctt ctcccacctg ttcgactccc catccccagg atgtcaacct 180

cagtccctca aggccatacc tggacccaac gggtgaagaa agacgatgag gaggaggacc 240

cgctggacca gctga 255


<210> 100
<211> 255
<212> DNA
<213> Homo Sapiens

<400> 100
gaatcgtcga cccacgcgtc cgcccacgcg tccggcagag cttccgggct gcgctcttcg 60

ttgcccagtt tccgctcagt ggtcgcgtct ccgcccccca cccaccagtc ccgctgcatt 120

ctcggccggg ctctaggcgc catggctccc cgcgggagga agcgtaaggc tgaggccgcg 180

gtggtcgccg tagccgagaa gcgagagaag ctggcgaacg gcggggaggg aatggaggag 240

gcgaccgttg ttatc 255


<210> 101
<211> 255
<212> DNA
<213> Homo Sapiens

<400> 101
gaatcgtcga cccacgcgtc cgcccacgcg tccgcaggtg caatcgtgga ataccacggg 60

cctggtgtag actccatctc ctgcactggc atggcgacaa tctgcaacat gggtgcagaa 120

attggggcca ccacttccgt gttcccttac aaccacagga tgaagaagta cctgagcaag 180

accggccggg aagacattgc caatctagct gatgaattca aggatcactt ggtgcctgac 240

cctggctgcc attat 255

<210>  102
<211>  255
<212>  DNA
<213>  Homo Sapiens

<400>  102
gaatcgtcga cccacgcgtc cggccgggcg cctcttgcaa tggagacggt catttcttca      60

gatagctccc cagctgtgga aaatgagcat cctcaagaga ccccagaatc caacaatagc     120

gtgtatactt ccttcatgaa gtctcatcgc tgctatgacc tgattcccac aagctccaaa     180

ttggttgtat ttgatacgtc cctgcaggtg aagaaagctt tttttgcttt ggtgactaac     240

ggtgtacgag ctgcc                                                      255


<210>  103
<211>  255
<212>  DNA
<213>  Homo Sapiens

<400>  103
gaatcgtcga cccacgcgtc cgaggcagtc agcgaggccc aggagaaatt gacagaatcc      60

aaccagaagc tggggctgct gcgggaggct ctggagcgga gacttgggga gctgcccgcc     120

gaccacccca aggggcggct gctgcgagaa gagctcgctg cggcctcctc cgctgccttc     180

agcacccgcc tggccgggcc ctttcccgcc acgcactaca gcaccctgtg caagcccgcg     240

ccgctcacag ggacc                                                      255


<210>  104
<211>  255
<212>  DNA
<213>  Homo Sapiens

<400>  104
gaatcgtcga cccacgcgtc cgcccacgcg tccgattctg gagaagccgg tggacaagca      60

ggacgcctac aggatgctgt cccggttgag tgggagagaa cacagcgtgt tcacaggtgt     120

cgcgatcgtc cactgctcca gcaaagacca tcagctggac accagggtct cggaattcta     180

cgaggaaacg aaggtgaagt tctcggagct gtccgaggag ctgctctggg aatacgtcca     240

cagcggggag cccat                                                      255


<210>  105
<211>  255
<212>  DNA
<213>  Homo sapiens

<400>  105
gaatcgtcga cccacgcgtc cgccgagctg ggatcggccc ccgggcgggg gcggtgcgag      60

cggcgccaag cagatcttag gggcggggac ggagccgggg cgggcgggac tgaagcggag     120

cccgggaacg gggcgggagg tcccagggtc ccgggttggg ggggtggagc agcatttcgt     180

cgccgcgggg gtgccgggac tccggccgca gtgtcgccgc catcacggac ttcctgtggg 240

acaagcgcac gggcc 255

<210> 106
<211> 255
<212> DNA
<213> Homo Sapiens

<400> 106
gaatcgtcga cccacgcgtc cgggaatcct atcaaaattg gttatggtaa agctacaccc 60

accacccgcc tctgggtggg aggcctggga ccttgggttc ctcttgctgc cctggcacga 120

gaatttgatc gatttggcac catacgcacc atagactacc gaaaaggtga tagttgggca 180

tatatccagt atgaaagcct ggatgcagcg catgctgcct ggacccatat gcggggcttc 240

ccacttggtg gccca 255

<210> 107
<211> 255
<212> DNA
<213> Homo Sapiens

<400> 107
agaatcgtcg acccacgcgt ccgcccacgc gtccgcggac gcgtgggctc cacggccgaa 60

gaatgtgatc tttgaagatg aggagaaatc caagatgctg gcccgcctgc tgaagagctc 120

ccatcccgaa gacctccgcg cagccaataa gctcatcaaa gagatggtgc aggaggacca 180

gaagcggatg gagaagatct cgaagagggt gaatgccatc gaggaggtga acaacaatgt 240

gaaactgctc acgga 255

<210> 108
<211> 255
<212> DNA
<213> Homo Sapiens

<220>
<221> misc_feature
<222> (9)..(9)
<223> n is a, c, g, or t

<220>
<221> misc_feature
<222> (14)..(14)
<223> n is a, c, g, or t

<220>
<221> misc_feature
<222> (50)..(50)
<223> n is a, c, g, or t

<220>
<221> misc_feature
<222> (63)..(63)

<223>  n is a, c, g, or t

<400>  108
gaatcgtcna cccncgcgtc cgggaagaac tggagaaaag gaaggatgan attgaacgag      60

aanttctccg aagggtggag gaagccaaac gcatcatgga aaagcagttg ctcgaagaac     120

tcgagcgaca gagacaagct gagcttgccg cacaaaaagc tagagagagg aagctggcgc     180

gtatggcagc cgaggagcac actctgcagg acactggaca agacagagga agaacgtgca     240

aaacgtgagg agcta                                                      255


<210>  109
<211>  255
<212>  DNA
<213>  Homo Sapiens

<400>  109
gaatcgtcga cccacgcgtc cgccatccgg gatgctaacc tgtacgtcag cgggctcccc      60

aagaccatga gccagaaaga gatggagcag ctcttctccc agtacggccg catcatcacg     120

tcccgcatcc tggtggacca ggtcacaggt gtctctcggg gtgtgggatt catccgcttt     180

gacaagagga ttgaggccga agaggctatc aaaggactga atgggcagaa gccgctgggc     240

gcagctgagc ccatc                                                      255


<210>  110
<211>  255
<212>  DNA
<213>  Homo Sapiens


<220>
<221>  misc_feature
<222>  (31)..(31)
<223>  n is a, c, g, or t

<220>
<221>  misc_feature
<222>  (40)..(40)
<223>  n is a, c, g, or t

<220>
<221>  misc_feature
<222>  (47)..(47)
<223>  n is a, c, g, or t

<220>
<221>  misc_feature
<222>  (53)..(53)
<223>  n is a, c, g, or t

<220>
<221>  misc_feature
<222>  (56)..(56)
<223>  n is a, c, g, or t

<220>

<221>  misc_feature
<222>  (61)..(61)
<223>  n is a, c, g, or t

<220>
<221>  misc_feature
<222>  (76)..(76)
<223>  n is a, c, g, or t

<220>
<221>  misc_feature
<222>  (98)..(98)
<223>  n is a, c, g, or t

<220>
<221>  misc_feature
<222>  (104)..(104)
<223>  n is a, c, g, or t

<220>
<221>  misc_feature
<222>  (106)..(106)
<223>  n is a, c, g, or t

<220>
<221>  misc_feature
<222>  (110)..(110)
<223>  n is a, c, g, or t

<220>
<221>  misc_feature
<222>  (112)..(112)
<223>  n is a, c, g, or t

<220>
<221>  misc_feature
<222>  (121)..(121)
<223>  n is a, c, g, or t

<220>
<221>  misc_feature
<222>  (145)..(145)
<223>  n is a, c, g, or t

<220>
<221>  misc_feature
<222>  (147)..(148)
<223>  n is a, c, g, or t

<220>
<221>  misc_feature
<222>  (150)..(150)
<223>  n is a, c, g, or t

<220>
<221>  misc_feature
<222>  (153)..(153)
<223>  n is a, c, g, or t

<220>
<221>  misc_feature
<222>  (156)..(156)
<223>  n is a, c, g, or t

<220>
<221>  misc_feature
<222>  (161)..(162)
<223>  n is a, c, g, or t

<220>
<221>  misc_feature
<222>  (169)..(169)
<223>  n is a, c, g, or t

<220>
<221>  misc_feature
<222>  (181)..(182)
<223>  n is a, c, g, or t

<220>
<221>  misc_feature
<222>  (184)..(184)
<223>  n is a, c, g, or t

<220>
<221>  misc_feature
<222>  (202)..(202)
<223>  n is a, c, g, or t

<220>
<221>  misc_feature
<222>  (215)..(215)
<223>  n is a, c, g, or t

<220>
<221>  misc_feature
<222>  (221)..(221)
<223>  n is a, c, g, or t

<220>
<221>  misc_feature
<222>  (229)..(229)
<223>  n is a, c, g, or t

<220>
<221>  misc_feature
<222>  (239)..(239)
<223>  n is a, c, g, or t

<400>  110
gaatcgtcga cccacgcgtc cggggagggg ngcccgaggn cgttgcngac ctnggngcta      60

nagccgctgc tgcctnggaa gacagatcct ccagtacncg cctncngtcn gngcagagtg     120

ncgtgtcctc ctcccacccc tagcngnncn ccntcncgga nngatttcnt aaacggcctt     180

nngncactag tcgatccgag cngccgccat ccgtnactgc ntgggttgnt gacactccng     240

ttggcaaggt ccact                                                      255


<210>  111
<211>  255
<212>  DNA
<213>  Homo Sapiens

<220>
<221>  misc_feature
<222>  (111)..(111)
<223>  n is a, c, g, or t

<220>
<221>  misc_feature
<222>  (124)..(124)
<223>  n is a, c, g, or t

<220>
<221>  misc_feature
<222>  (160)..(160)
<223>  n is a, c, g, or t

<400>  111
gaatcgtcga cccacgcgtc cgccttgagg gaagaagaaa ggagagaatc tgctgcagca      60

gacgctggct ttgctctctt tgagaagaag ggagccttag ctgaaaggca ncagctcatc     120

ccanaattgg tgcgaaatgg agatgaggag aatcccctcn aaaggggtct ggttgctgct     180

tacagtggtg acagtgacaa tgaggaggag ctggtggaga gacttgagag tgaggaagag     240

aagctagctg actgg                                                      255


<210>  112
<211>  255
<212>  DNA
<213>  Homo Sapiens

<400>  112
gaatcgtcga cccacgcgtc cgtgaaacca cagcctacgc cctgctgcac ctcctgcttc      60

acgagggcaa agcagagatg gcagaccagg ctgcggcctg gctcacccgt cagggcagct     120

tccaaggggg attccgcagt acccaagaca cggtgattgc cctggatgcc ctgtctgcct     180

actggattgc ctcccacacc actgaggaga ggggtctcaa tgtgactctc agctccacag     240

gccggaatgg gttca                                                      255


<210>  113
<211>  255
<212>  DNA
<213>  Homo Sapiens

<400>  113
gaatcgtcga cccacgcgtc cggcgtgttt cactgctgca ccttctgctc cagcgggggc      60

cagaaaaccg ctcgctgcgc ctgtggaggc accatgccag gtgggtacct aggctgtggc     120

catggacaca aaggccaccc aggtcatccc cactggtcat gctgtggaaa atttaatgag     180

aaatctgaat gcacatggac aggtgggcag agcgcaccga ggagtctact taggactgtg     240

gctctctgat gggtt                                                      255


<210>  114

<211>  255
<212>  DNA
<213>  Homo Sapiens

<400>  114
gaatcgtcga cccacgcgtc cgaggacccc caccttcgct cctgggagcc ccgctgcacc      60

ccaggagttc cattccagcc tccccagtgc gagtgcggcc gagcgctgac ttttgggggc     120

tccctccccc gtctcactgg gtccctctgc ggctcccccg cccgctgtcc cggttcagac     180

gtggctgcac cccggagggg ggcgccggcc gagacctgag acgtggcgct ccctctctgc     240

cggcctcggc ttcag                                                      255


<210>  115
<211>  255
<212>  DNA
<213>  Homo Sapiens

<400>  115
gaatcgtcga cccacgcgtc cgacgccatc ctggtgctgg cgcccaaagg ctccctgggg      60

gaccgcttct gccgcgaccg cctgcccctc ctggacattg cttgcaacaa gttcctgacc     120

tgcagcgtgg aggatgggga gctggtcttc cgccacgccc aggacctcat cctggagatc     180

atctacactg agcccgtcga cctgtccctg ggcaccctgg gggagatcag tgggcaccag     240

ctcatgagtc tgtct                                                      255


<210>  116
<211>  255
<212>  DNA
<213>  Homo Sapiens

<400>  116
gaatcgtcga cccacgcgtc cgggcatcac gaaacattgg atcatgacat gtcgggcgat      60

gcttggaaga gcccagcatg tatgtatgca cacattgtgt gtgtgggaag gacaaagcca     120

ctctcacaag aaagggcacc aggactgctc tccaaggaac tggacctgtc cagacagtta     180

cactccaagg tcattggaga gaacttctgt atgggcaagc ctgagaggga gaggaaacaa     240

aagctgtgtc ctggc                                                      255


<210>  117
<211>  255
<212>  DNA
<213>  Homo Sapiens

<400>  117
gaatcgtcga cccacgcgtc cggagctttc cttctttgtc gtttttcttg tggatttatc      60

tttcttttta tgtttggcag cttgatgatc acatttagtt tgactccttt gtacccacac     120

tgggagaagc aaagaagaac acttggcctt aaaggatggg caaggctggg cacagtggcc     180

cacacctgga tcccagcctt agaggatggg cgaggccggg cacagtggcc cacacctgga     240

tcccagcctt agagg                                                    255


<210>  118
<211>  255
<212>  DNA
<213>  Homo Sapiens

<400>  118
gaatcgtcga cccacgcgtc cgcaggagag gctcagccta gactatacag tgactccggg      60

gagcaccaga aaagaccctg cccaggcccc acccagacca caacatccac ctctctggtg     120

gggccagtgt gtggtggggg cctcccgtta gaggtagatg tgtgcatgcc tggtgcaggg     180

ggctggtgca gagcattggc aggagtggca caggaggccg gcggcacctg gagctccgca     240

gcatccaccc aggcc                                                    255


<210>  119
<211>  255
<212>  DNA
<213>  Homo Sapiens

<400>  119
gaatcgtcga cccacgcgtc cgcggagatc ctgcgctccg gcaagcggcc agacctagtg      60

tcctacgtgc agactctgtg caagggtctg tcgcagccca ccaccaatct ggtggccggc     120

tgtctgcagc tcaactctcg caacttcctc acggagcaag gcgccgacgg tgccggccgc     180

ttccacggct cgggcggccc gttcgccatg cacccctacc cgtacccgtg ctcgcgcctg     240

gcgggcgcac agtgc                                                    255


<210>  120
<211>  255
<212>  DNA
<213>  Homo Sapiens

<400>  120
gaatcgtcga cccacgcgtc cgcccacgcg tccgctccag accacagaag gggagtcctg      60

gggacaggac tggtgtagac aggcatctcc accgccccgg ttagccagta cctaacaccc     120

actcctgccc tcggccgatg tggcaaaccg gggaggcttt tgtgtggggc cggggctgcg     180

gccatggcag ccaccctcca gctcccgggg gctggggaag acgctggggt ccccgtggag     240

gctggagcgg cgttc                                                    255


<210>  121
<211>  255
<212>  DNA
<213>  Homo Sapiens

<400>  121
gaatcgtcga cccacgcgtc cgggtctaca ccgacttcga cggcacccga gtgtacagcc      60

ccccggagtg gatccgctac caccgctacc acgggcgctc ggccaccgtg tggtcgctgg 120

gcgtgcttct ctacgatatg gtgtgtgggg acatcccctt cgagcaggac gaggagatcc 180

tccgaggccg cctgctcttc cggaggaggg tctctccaga gtgccagcag ctgatccggt 240

ggtgcctgtc cctgc 255

<210> 122
<211> 255
<212> DNA
<213> Homo Sapiens

<220>
<221> misc_feature
<222> (39)..(39)
<223> n is a, c, g, or t

<220>
<221> misc_feature
<222> (56)..(56)
<223> n is a, c, g, or t

<220>
<221> misc_feature
<222> (112)..(112)
<223> n is a, c, g, or t

<220>
<221> misc_feature
<222> (135)..(135)
<223> n is a, c, g, or t

<220>
<221> misc_feature
<222> (163)..(163)
<223> n is a, c, g, or t

<400> 122
gaatcgtcga cccacgcgtc cgcccacgcg tccgcccang cgtccggagc catggntcag 60

aaaatggact gtggtgcggg cctcctcggc ttccaggctg aggcctccgt anaagacagc 120

gccttgctta tgcanacctt gatggaggcc atccagatct canaggctcc acctactaac 180

caggccaccg cagctgctag tccccagagt tcacagcccc caactgccaa tgagatggct 240

gacattcagg tttca 255

<210> 123
<211> 255
<212> DNA
<213> Homo Sapiens

<400> 123
gaatcgtcga cccacgcgtc cggaagccct gaaggagcag gaggagatca acttccggct 60

gaggcagtac atggacaaga ttatcctcgc catcctggac cacaatccct ccatcctcga 120

gatcaaacac taaggcacgg ggctggctgc agagcagcct taggaccctg ggaccaaggg 180

cagaccctgc ccaaggatgc aggcctaagc cgggcctcac actcacactg taaatgtctc 240

tctggccacc atgcg 255

<210> 124
<211> 255
<212> DNA
<213> Homo Sapiens

<400> 124
gaatcgtcga cccacgcgtc cggtcaggta gactgtgagg agaaagtgaa gggttttaag 60

tggggaaatg acatgatttg gtttttattt taggattaca ggcgtgagcc gccgcgcctg 120

gctgcttgca gcctttatat tatctatggc tgctattata taccctctcc agttctgctg 180

cagtggcata atagagtaat tgtgccgaga atgaatttgt ctctaggccc aaaagcctaa 240

aatatctaca ttctg 255

<210> 125
<211> 255
<212> DNA
<213> Homo Sapiens

<400> 125
taatcgtcga cccacgcgtt ccgaccagca gaatgccgcg tgcgagtggc tgctggggga 60

ccggaagccc tctccggagg agctggacaa gggcatcgac cccgacagtc ctctctttca 120

ggccatcctg gataacccgg tggtgcagct gggcctgacc aacccgaaaa cattgctagc 180

atttgaagac atgctggaga acccactgaa cagcacccag tggatgaatg atccagaaac 240

ggggcctgtc atgct 255

<210> 126
<211> 255
<212> DNA
<213> Homo Sapiens

<400> 126
gaatcgtcga cccacgcgtc cgccagcccc cgcgcccgcc cgccagcccg cccagcggtc 60

gggtccgggc gcccgcgcag aatcagctgt ctgagctgcc caggcggcgg gggagcagcg 120

agcgggcttc agcgagccgc aggaggcaca ggcctgtcct gggtccccgc aggtctgcgc 180

gtctgttgtt cccagcgctc tgagaggcct gaaaaggaag agcaacctgt ccagaatccc 240

cgcaggaaag gaaaa 255

<210> 127
<211> 255
<212> DNA
<213> Homo Sapiens

<400> 127

gaatcgtcga cccacgcgtc cggacgccat gacgccccgg ctggtgtgtg tcggtgtgta 60

tgtgtgtgtg tgagtgtgcg cgctccgagt gtgtgtgtat ttgtgtatcg gcggtcccgc 120

aggtcccgga tgttgcggac agtatgaggc aagcgcaggg ggcggggacc agcagctgtc 180

gccgccgctc tcaggctctg ggaaccaccc ttctactttc tgtctctagg aatttcacta 240

ctctagggtg aagag 255

<210> 128
<211> 255
<212> DNA
<213> Homo Sapiens

<400> 128
gaatcgtcga cccacgcgtc cggtcggtgt gtatgtgtgt gtgtgagtgt gcgcgctccg 60

agtgtgtgtg tatttgtgta tcggcggtcc cgcaggtccc ggatgttgcg gacagtatga 120

ggcaagcgca gggggacggg gaccagcagc tgtcgccgcc gctctcaggg tgaagaggga 180

acagaaatct ttgccccctg actttggaaa tctcgtttaa ccttcaaact ggcgatgtca 240

agggttccaa gtcct 255

<210> 129
<211> 255
<212> DNA
<213> Homo Sapiens

<400> 129
gaatcgtcga cccacgcgtc cggtgggagt gcgggtgggg gtgtggccga gccccggcag 60

gaagccccgc ccagacggtg ttcagggaac ccggagccca agcgctccgg cggagcccaa 120

aagggtgggg gtgggagggg cagaggccaa cggatccccc tgcctgtcgc accccttggc 180

gggagacggg aaggcagcgg gctgcgtacg atgggaccct ggtgcagacg ccgggccggc 240

tgacatttgg acccc 255

<210> 130
<211> 255
<212> DNA
<213> Homo Sapiens

<400> 130
gaatcgtcga cccacgcgtc cgcgggaggg cagggccgac ggacggcggg cggcggcggc 60

ggtggcggcg ctggagtcgg cgcgggtgct ggcgccatgg aggccgagcg gggtcccgag 120

cgccggcctg cggagcgtag cagcccgggc cagacgccgg aggagggcgc gcaggccttg 180

gccgagttcg cggcgctgca cggcccggcg ctgcgcgctt cgggggtccc cgaacgttac 240

tggggccgcc tcctg 255

<210> 131

<211>  255
<212>  DNA
<213>  Homo Sapiens

<400>  131
agaatcgtcg acccacgcgt ccgttgagag cttgggctcc tccgtgagcc gccggcgcgc      60

ttcgctgcct ggcggccttt ccccgagccg agcccgcttc ccaaggccgc cagtcgtcgc     120

ctgcccgcgc ctgcgcagcg tcccgggccg agagggccac ggcggcggcc atggcgcacc     180

gctgtttgcg gctgtggggc cggggcggct gctggccccg cggcctacag cagctcctcg     240

tgcctggcgg cgtgg                                                      255


<210>  132
<211>  255
<212>  DNA
<213>  Homo Sapiens

<400>  132
gaatcgtcga cccacgcgtc cgccggaccg ggacgcagag tctgcggacc cggcgccgag      60

gcggccaccc gagacgcggc gcgcacgctc cggcctgcgc ccggcccggc catggcggcc     120

ccccgcccgt ctcccgcgat ctccgtttcg gtctcggctc cggcttttta cgccccgcag     180

aagaagttcg gccctgtggt ggccccaaag cccaaagtga atcccttccg gcccggggac     240

agcgagcctc ccccg                                                      255


<210>  133
<211>  255
<212>  DNA
<213>  Homo Sapiens

<400>  133
gaatcgtcga cccacgcgtc cgccgaaggg ccgggacctg gctcagggga cgaagccaat      60

gggccccggg gagagaggca gacccggact cggggcccgg ccccatctgc aatgccccag     120

agcaggtcta ccgagtcagc ccatgctgcc accctgccgc ctcgaggccc agagccatct     180

gcccaggagc agatggaggg gatgctgtgc cgcaagcagg agatggaggc cttcgggaag     240

aaggctgcca acagg                                                      255


<210>  134
<211>  255
<212>  DNA
<213>  Homo Sapiens

<400>  134
gaatcgtcga cccacgcgtc cggcaaccca ggcagccacg gctgtttcgg agctcaggac      60

tctaaaatgg cagagcagct ttctccagga aaggcggtgg atcaggtgtg caccttcctt     120

ttcaaaaagc ctgggcggaa aggggctgct ggacgcagaa agcgcccggc ctgcgaccca     180

gagcccggag aaagcggcag cagtagcgac gaaggctgca ctgtggttcg accggaaaag     240

aagcgggtga cccac 255

<210> 135
<211> 255
<212> DNA
<213> Homo Sapiens

<400> 135
gaatcgtcga cccacgcgtc cgggccttgc cccgctctgc agcacagaca ggccagatgc 60

atttgtcctt tgcctagcta ctccccaggt agagagtgct cctggtggcc tggcaggtct 120

gggcccttct ctccctgccc aggttgtccc tggagggcag ccctcactcc ctttggggga 180

gaggcagaca ttgctgccca cagacctgcc tctgactcaa ctgtgtccac cctccctggt 240

ccctaccccc aagtc 255

<210> 136
<211> 255
<212> DNA
<213> Homo Sapiens

<400> 136
gaatcgtcga cccacgcgtc cgagttcaag cagctggctc tggaggacgc caaagaaggc 60

tacagatatg gtttggagtg cctttttcga tactacagtt atggcctgga aaagaagttc 120

cggctggaca tattcaagga ttttcaggag gaaacggtga aggactatga agctggccaa 180

ctgtatgggc tggagaagtt ctgggccttc ttgaaatatt ccaaagccaa aaatttggac 240

attgacccca aactg 255

<210> 137
<211> 255
<212> DNA
<213> Homo Sapiens

<400> 137
gaatcgtcga cccacgcgtc cgctcctcca gtgctgtgcg catggcagtg ggctgcctgc 60

tggagctggc cttcaaggtg gctgcaggag agctcaagaa tggatttgcc atcatccggc 120

ccccaggaca ccacgccgag gaatccacag ccatgggatt ctgcttcttc aactctgtag 180

ccatcaccgc aaaactccta cagcagaagt tgaacgtggg caaggtcctc atcgtggact 240

gggacattca ccatg 255

<210> 138
<211> 255
<212> DNA
<213> Homo Sapiens

<400> 138
gaatcgtcga cccacgcgtc cggcaggtca gcagattcct ggctgcagct tggagggccc 60

cagactttgt gcctcgttac tgtaaactct atgagcactt gcagagagca ggctccgagc 120

tgtttgggcc tcgggcagcc ttcatgctgg ctctgcgcag tggcttctct ggcgccttgc 180

tgcagcagtc cttcctcact gctgctcaca tgagtgagca gtttgccagg tacattgacc 240

aacagatcca gggtg 255

<210> 139
<211> 255
<212> DNA
<213> Homo Sapiens

<220>
<221> misc_feature
<222> (61)..(61)
<223> n is a, c, g, or t

<400> 139
gaatcgtcga cccacgcgtc cgcacagagc tcagtgcaac caccagaagg agacagtgaa 60

ncaaaagctc ctgaagaatc atcagaggat gtgacaaaat atcaggaagg agtatctgca 120

gaaaacccag ttgagaacca tatcaatata acacaatcag ataagttcac agccaagcca 180

ttggattcca actcaggaga aagaaatgac ctcaatcttg atcgctcttg tggggttcca 240

gaagaatctg cttca 255

<210> 140
<211> 255
<212> DNA
<213> Homo Sapiens

<400> 140
gaatcgtcga cccacgcgtc cgcggaagcg ggggtgcagc gcggcagaat gagggttgat 60

tcctcggctg accccacaat gtcgcaggag caagggccgg ggtcctccac gcctcccagt 120

tctccgacac ttcttgacgc tctgctccag aacctttacg actttggagg tacagaaggt 180

gaaacagaac agaagaagat cataaagaaa agggaaaaca agaagagaga tgtgatggct 240

tcagcggcct tggca 255

<210> 141
<211> 255
<212> DNA
<213> Homo Sapiens

<400> 141
gaatcgtcga cccacgcgtc cgaccgcggg gaggctcacc tcgagctgaa cgccttccgc 60

aggaagcatg attgtgccct ggtcatctcg ggagacttcc tggaggtttg cctcaagtac 120

tatgagtacg agttcatgga gctggcctgc cagtgcccgg ccgtagtctg ctgccgatgt 180

gcccccaccc agaaggccca gatcgtgcgc ctgcttcagg agcgcacggg caagctcacc 240

tgtgcactca gtaaa                                                    255

<210>  142
<211>  255
<212>  DNA
<213>  Homo Sapiens

<400>  142
gaatcgtcga cccacgcgtc cgcgagaggc cagctcagac ctcccggctc gacaggcggc      60

gcgggcggcg gtaaaatgtc ggttccagga ccttaccagg cggccactgg gccttcctca     120

gcaccatccg cacctccatc ctatgaagag acagtggctg ttaacagtta ttaccccaca     180

cctccagctc ccatgcctgg gccaactacg gggcttgtga cggggcctga tgggaagggc     240

atgaatcctc cttcg                                                    255

<210>  143
<211>  255
<212>  DNA
<213>  Homo Sapiens

<220>
<221>  misc_feature
<222>  (103)..(103)
<223>  n is a, c, g, or t

<220>
<221>  misc_feature
<222>  (243)..(243)
<223>  n is a, c, g, or t

<400>  143
gaatcgtcga cccacgcgtc cgataagccc aggcccaact tgggatatca aacgggaaca      60

cgacgttagg aaaccaaagg agctttcagc ctgcggccag aanaagcagc gcctcgggga     120

gcagagggag cgctcggcga gtccgcagag ggccgcgcgg ccgaggctcg aggaggcgcc     180

cggcggccat gggcggcccg aggccggcag gcccgcctcg gaggccaggg cgcccgggct     240

cgnggcggca gacgc                                                    255

<210>  144
<211>  255
<212>  DNA
<213>  Homo Sapiens

<400>  144
gaatcgtcga cccacgcgtc cgataaatcc ctggggccag tgtcattcaa ggacgtggct      60

gtggacttca cccaggagga atggcagcag ctggatcctg agcagaagat aacttacagg     120

gatgtgatgc tggagaacta cagcaatcta gtttctgtgg ggtatcacat tatcaaaccg     180

gatgttatca gcaagttgga gcaaggagaa gagccatgga tagtagaagg agaattccta     240

cttcagagct atcca                                                    255

<210>  145
<211>  255
<212>  DNA
<213>  Homo Sapiens

<400>  145
gaatcgtcga cccacgcgtc cgagccgcca tcttgtggga gcaaaaccaa cgcctggctc      60

ggagcagcag cctctgaggt gtccctggcc agtgtccttc cacctgtcca caagcatggg     120

gaacatcttc gccaacctct tcaagggcct ttttggcaaa aaagaaatgc gcatcctcat     180

ggtgggcctg gatgctgcag ggaagaccac gatcctctac aagcttaagc tgggtgagat     240

cgtgaccacc attcc                                                      255


<210>  146
<211>  255
<212>  DNA
<213>  Homo Sapiens

<400>  146
gaatcgtcga cccacgcgtc cgccagcttc ctgccagacc gcgaccgcga ggaggaggag      60

aaccggctcc gagaggagct gcgccaagag tgggaggcgc agcgcgagaa agtgaaggac     120

gaggagatgg aggtcacctt cagctactgg gacggctcgg gccaccggcg cacggtgcgg     180

gtgcgcaagg gcaacacggt gcagcagttc ctgaagaagg cgctgcaggg gctgcgcaag     240

gacttcctgg agctg                                                      255


<210>  147
<211>  255
<212>  DNA
<213>  Homo Sapiens

<400>  147
gaatcgtcga cccacgcgtc cgaattgatt ccagacagca ttggaaaaga catagaaaag      60

gcttgccaat ctatttatcc tctccatgat gtcttcgtta gaaaagtaaa aatgctgaag     120

aagcccaagt ttgaattggg aaagctcatg gagcttcatg gtgaaggcag tagttctgga     180

aaagccactg gggacgagac aggtgctaaa gttgaacgag ctgatggata tgaaccacca     240

gtccaagaat ctgtt                                                      255


<210>  148
<211>  255
<212>  DNA
<213>  Homo Sapiens

<400>  148
gaatcgtcga cccacgcgtc cgcccacgcg tccgcccacg cgtccgccca cgcgtccgat      60

aagacggcgt cggtgttgca gtctgtgtcc ttggaggtga ccagggccac tgcaggcatg     120

gtgctagcag agctgtacgt ctctgaccga gagggaagcg atgccacggg agatggaacc 180

aaggagaaac catttaaaac aggtctaaag gctttgatga cagtagggaa agaaccattt 240

cctaccattt acgta 255

<210> 149
<211> 255
<212> DNA
<213> Homo Sapiens

<400> 149
gaatcgtcga cccacgcgtc cggtgaaggc ctcgttgaga gaaggtctca ttcggtgttt 60

tgggaagaga gtcgtgtggg cccaggtctg tctgctatca gctatgccgc tgcccgttgc 120

gctgcagacc cgcttggcca agagaggcat cctcaaacat ctggagcctg aaccagagga 180

agagatcatt gccgaggact atgacgatga tcctgtggac tacgaggcca ccaggttgga 240

gggcctacca ccaag 255

<210> 150
<211> 255
<212> DNA
<213> Homo Sapiens

<400> 150
gaatcgtcga cccacgcgtc cgctgagatg agcaacctgg acctaaccaa gattctgtcc 60

aagaaataca aggagcttcc ggagaagaag aagatgaaat atattcagga cttccagaga 120

gagaaacagg agttcgagcg aaacctggcc cgattcaggg aggatcaccc cgacctaatc 180

cagaatgcca agaaatcgga catcccagag aagcccaaaa ccccccagca gctgtggtac 240

acccacgaga agaag 255

<210> 151
<211> 255
<212> DNA
<213> Homo Sapiens

<400> 151
gaatcgtcga cccacgcgtc cgcggacgcg tggggtcgct gccgctgccg ctgccactgc 60

cactgccacc tcgcggatca ggagccagcg ttgttcgccc gacgcctcgc tgccggtggg 120

aggaagcgag agggaagccg cttgcgggtt tgtcgccgct gctcgcccac cgcctggaag 180

agccgagccc cggcccagtc ggtcgcttgc caccgctcgt agccgttacc cgcgggccgc 240

cacagccgcc ggccg 255

<210> 152
<211> 255
<212> DNA
<213> Homo Sapiens

<400>  152
gaatcgtcga cccacgcgtc cgctggagat ccaaagggat ttgcgtttgt ggaatttgaa      60

acaaaagaac aagcagcaaa agcaattgag tttcttaaca acccaccaga agaagcacca     120

agaaaacctg gcatatttcc taaaacagtg aaaaataagc ccattccagc cttaagagtt     180

gtggaagaga agaaaaagaa aaagaagaag aaaggccgaa tgaaaaagga agacaatatc     240

caagccaaag aagaa                                                      255


<210>  153
<211>  255
<212>  DNA
<213>  Homo Sapiens

<400>  153
gaatcgtcga cccacgcgtc cgggccgttg tcaccatgcc ggtcgtccgg aagattttcc      60

gtcgccgccg gggcgactcg gagtcagagg aagatgagca ggactcagag gaggttcgat     120

taaaactgga agagaccaga gaagtacaga acttgaggaa gaggcccaac ggggtgagtg     180

ctgtggcctt gctggtggga gagaaggtac aagaggagac cactctagtg gatgatccct     240

ttcagatgaa gacag                                                      255


<210>  154
<211>  255
<212>  DNA
<213>  Homo Sapiens

<400>  154
gaatcgtcga cccacgcgtc cggccgctgt gccccgcgcc gcatttctct ccccgctgct      60

tccccttctc ctgggcttcc tgctcctctc cgctccgcat ggcggcagcg gcctgcacac     120

caagggcgcc cttcccctgg atacggtcac tttctacaag gtcattccca aaagcaagtt     180

cgtcttggtg aagttcgaca cccagtaccc ctacggtgag aagcaggatg agttcaagcg     240

tcttgctgaa aactc                                                      255


<210>  155
<211>  255
<212>  DNA
<213>  Homo Sapiens

<400>  155
gaatcgtcga cccacgcgtc cgagaaagtc atgtataagg gactcgtccc cgaggataaa      60

acattgagag aaataaaagt gaccagtggg gccaagatca tggtggttgg ctccaccatc     120

aatgatgttt tagcagtaaa cacacccaaa gatgctgcgc agcaggatgc aaaggccgaa     180

gagaacaaga aggagcctct ctgcaggcag aaacaacaca ggaaagtgtt ggataaagga     240

aaacctgaag atgtg                                                      255

<210> 156
<211> 255
<212> DNA
<213> Homo Sapiens

<400> 156
gaatcgtcga cccacgcgtc cgcccacgcg tccgcgctga cgggcacgct gactggaggc 60

tggcggacag gcgacagcga cctgcggcag gaccagagga gcgagagcag caagaaccac 120

acccagcagc aatgtcagcg gaagtggaaa cctcagaggg ggtagacgag tcagaaaaaa 180

agaactctgg ggccctagaa aaggagaacc aaatgagaat ggctgacctc tcggagctcc 240

tgaaggaagg gacca 255


<210> 157
<211> 255
<212> DNA
<213> Homo Sapiens

<400> 157
gaatcgtcga cccacgcgtc cgccttcagg agccctgagc ccaacatcca tgagacaccc 60

cgagttgtgc ttcatgagag ttggattctc tgcccactct ttcagggatg tccaaggtcc 120

atgttagggg gcaggccccc agctccactc actgcacaag caccctaaat gttggaagca 180

caaaatggtt gtgatttgag cacaatggca gggtcccgca ggcagacttg acaggcccag 240

gcccgggagg atccc 255


<210> 158
<211> 255
<212> DNA
<213> Homo Sapiens

<400> 158
gaatcgtcga cccacgcgtc cggccgaagc ggctgcatct ggcgccgcgt ctgccccgcg 60

tgctcggagc ggattctgcc cgccgtcccc ggagccctcg gcgccccgct gagcccgcga 120

tcacttcctc cctgtgacca accggcgctg caggttagag cctggcaatg ccgtttgggt 180

gtgtgactct gggcgacaag aagaactata accagccatc ggaggtgact gacagatatg 240

atttgggaca ggtca 255


<210> 159
<211> 255
<212> DNA
<213> Homo Sapiens

<400> 159
gaatcgtcga cccacgcgtc cggcacagtc tccagagatg aagtgagaag gaaaaaagca 60

gtttataaga aggctgaact tgctaaaaaa acagaagttc aggcccactc tccctccagg 120

aaattcattt taaaacctgc tatcaaatat actagaccaa ctcatctctc ctgtgttaag 180

cggaaaacca cagcagcagg tggggaatca gctctggctc ccagtgtatt taaacaggca 240

aaggacaaag tctct 255

<210> 160
<211> 255
<212> DNA
<213> Homo Sapiens

<400> 160
gaatcgtcga cccacgcgtc cgcccacgcg tccggagagc gcctgcctgt gcgccccgag 60

cggggctggg actcttccaa gatgcccacg ttcgcacaga gaccccggat cgcggaagct 120

cgcgtctcga aaggcctgcg gtctcacgcc ctgcccgtcc tgggttcacg gtttttcatc 180

acctgcggct gtcctgcgat cgaccacagc tgtgcaggag gggcaggagg tatctgttgc 240

tgcagttacc ggaac 255

<210> 161
<211> 255
<212> DNA
<213> Homo Sapiens

<400> 161
gaatcgtcga cccacgcgtc cgctaccctt aaaggacaaa gatttaccaa cactgaagac 60

ccttacaagt tgacagtttc taaagagggt tctaggaatg ttctgtgtaa ggtcagcttc 120

actggaataa acatacatct tcccaaggtg actacttcaa aggcaacact acccattcgg 180

gtgcagaaat taccgtactt actctgccat aaatgtaaaa tgtgtataaa gttaagctat 240

tgcatttttt cataa 255

<210> 162
<211> 255
<212> DNA
<213> Homo Sapiens

<400> 162
gaatcgtcga cccacgcgtc cgatctgccc catctgcttc aagaacgaga tggacttctg 60

gaaggtggac ctcaagttcc tggacgactg ttgcaagagc cacctgagcg agaagcgcga 120

ggagctggag gagatcgcgc gccgcgtgca gctcatcctg gacgacctgg gcgtggacgc 180

ggccgagggc cgctggcgcc gctgccagaa gtgcgtctgg aagttcctgg agaagcccga 240

gtcgtcgtgc ccggc 255

<210> 163
<211> 255
<212> DNA
<213> Homo Sapiens

<220>

<221>  misc_feature
<222>  (51)..(51)
<223>  n is a, c, g, or t

<220>
<221>  misc_feature
<222>  (173)..(174)
<223>  n is a, c, g, or t

<220>
<221>  misc_feature
<222>  (176)..(176)
<223>  n is a, c, g, or t

<220>
<221>  misc_feature
<222>  (182)..(182)
<223>  n is a, c, g, or t

<220>
<221>  misc_feature
<222>  (237)..(237)
<223>  n is a, c, g, or t

<400>  163
gaatcgtcga cccacgcgtc cgcgattggt gtcctgtcac catggcgctg nctgtcttgc      60

gggtcctgga gccctttccg accgagacac ccccgttggc agtgctgctg ccacccgggg     120

gcccgtggcc ggcggcggag ctgggcctgg tgctggccct gaggcctgca ggnnanagcc     180

cngcagggcc ggcgctgctg gtggcagccc tggaggggcc ggacgcgggc accgaanagc     240

agggtcccgg gccgc                                                      255


<210>  164
<211>  255
<212>  DNA
<213>  Homo Sapiens

<400>  164
gaatcgtcga cctgcaagat ctgcggccgc tccctatagt gagtcgtatt aagcttaatt      60

agctgagctt ggactcctgt tgatagatcc agtaatgacc tcagaactcc atctggattt     120

gttcagaacg ctcggttgcc gccgggcgtt ttttattggt gagaatccaa gctagcttgg     180

cgagattttc aggagctaag gaagctaaaa tggagaaaaa aatcactgga tataccaccg     240

ttgatatatc ccaat                                                      255


<210>  165
<211>  255
<212>  DNA
<213>  Homo Sapiens

<400>  165
gaatcgtcga cccacgcgtc cgggtagact caaagaggtg tgaggtcaga gcagacattt      60

gagggggcag catccctttg ggacctcggc ccctgctccc ctttacccac tggtggaggc     120

gggaggttgg gtgagagttg ggtgtatgtt ttctgccctg tccacctgtg caccaggcca 180

gggacacctt caacaatgcc tgctgtgcca gtgtttgtgt gggtcactga ccctgtgtac 240

cagactcact tggta 255

<210> 166
<211> 255
<212> DNA
<213> Homo Sapiens

<400> 166
gaatcgtcga cccacgcgtc cggcggccgt acccatggtg gaggcctttg ctcttcttgt 60

ggcgtgctgg ggtgggcagg tgggctgcag ccccagcacc agcctgctcc agctcccaca 120

ccgcccacag acctccctgt cctcatattc tctgaccgca ggcctgcctg cctctgctct 180

gctccgcccc agaggggctg ggttggacct ggtgttcaag gccattccta tctgcccccc 240

agcccactgc tggag 255

<210> 167
<211> 255
<212> DNA
<213> Homo Sapiens

<400> 167
gaatcgtcga cccacgcgtc cgggccagcc ccttgtcctc tgccttctgc tggcagagga 60

gcagctggac tggggccttt tggcacagca gccggtgtct cctgcgcccg cctcccccat 120

ggccccatgc agccccaggg gcttcccccc tgcccatgga gtagagcccg agatcctggc 180

cactatgcca gttctgacct cgcatccccc taccccgagc ccatgcagtc tgggaacatg 240

ccgccttctc tccag 255

<210> 168
<211> 244
<212> DNA
<213> Homo Sapiens


<220>
<221> misc_feature
<222> (1)..(1)
<223> n is a, c, g, or t

<220>
<221> misc_feature
<222> (19)..(19)
<223> n is a, c, g, or t

<220>
<221> misc_feature
<222> (33)..(33)
<223> n is a, c, g, or t

<220>

<221>  misc_feature
<222>  (43)..(44)
<223>  n is a, c, g, or t

<220>
<221>  misc_feature
<222>  (58)..(58)
<223>  n is a, c, g, or t

<220>
<221>  misc_feature
<222>  (70)..(70)
<223>  n is a, c, g, or t

<220>
<221>  misc_feature
<222>  (74)..(74)
<223>  n is a, c, g, or t

<220>
<221>  misc_feature
<222>  (96)..(96)
<223>  n is a, c, g, or t

<220>
<221>  misc_feature
<222>  (102)..(102)
<223>  n is a, c, g, or t

<220>
<221>  misc_feature
<222>  (118)..(118)
<223>  n is a, c, g, or t

<220>
<221>  misc_feature
<222>  (147)..(147)
<223>  n is a, c, g, or t

<220>
<221>  misc_feature
<222>  (155)..(155)
<223>  n is a, c, g, or t

<220>
<221>  misc_feature
<222>  (162)..(162)
<223>  n is a, c, g, or t

<220>
<221>  misc_feature
<222>  (169)..(169)
<223>  n is a, c, g, or t

<220>
<221>  misc_feature
<222>  (180)..(180)
<223>  n is a, c, g, or t

<220>
<221>  misc_feature
<222>  (183)..(183)
<223>  n is a, c, g, or t

<220>
<221>  misc_feature
<222>  (187)..(187)
<223>  n is a, c, g, or t

<220>
<221>  misc_feature
<222>  (190)..(190)
<223>  n is a, c, g, or t

<220>
<221>  misc_feature
<222>  (197)..(197)
<223>  n is a, c, g, or t

<220>
<221>  misc_feature
<222>  (201)..(201)
<223>  n is a, c, g, or t

<220>
<221>  misc_feature
<222>  (208)..(208)
<223>  n is a, c, g, or t

<220>
<221>  misc_feature
<222>  (211)..(211)
<223>  n is a, c, g, or t

<220>
<221>  misc_feature
<222>  (221)..(221)
<223>  n is a, c, g, or t

<220>
<221>  misc_feature
<222>  (223)..(223)
<223>  n is a, c, g, or t

<220>
<221>  misc_feature
<222>  (227)..(227)
<223>  n is a, c, g, or t

<220>
<221>  misc_feature
<222>  (230)..(230)
<223>  n is a, c, g, or t

<220>
<221>  misc_feature
<222>  (232)..(232)
<223>  n is a, c, g, or t

<220>
<221>  misc_feature
<222>  (235)..(235)
<223>  n is a, c, g, or t

<220>
<221>  misc_feature

<222>  (238)..(239)
<223>  n is a, c, g, or t

<220>
<221>  misc_feature
<222>  (243)..(243)
<223>  n is a, c, g, or t

<400>  168
naatcgtcga cccacgcgnt ccggccagtc cgnccgtccg gannccggct cgctgggnca      60

gcatggcggn gtcnccgctg ctctgggggc cgcggntcgg gngcatccgc cttttggngc     120

tgcgtgctgc tcggcctgtt tcggccnacc cccgngctct gngcgcggnc ggtaaatgan     180

ccncgcngcn taaacgnaag ngtctccncc ngttggctga nantggngcn cntcnccnnt     240

tcnt                                                                  244


<210>  169
<211>  255
<212>  DNA
<213>  Homo Sapiens

<400>  169
gaatcgtcga cccacgcgtc cggtgagaac cgagccacag tcatgtttcc tacatcagcc      60

tcctgctcag gggtttgtac agagaccact gccagccctc taggtggtcc ctgcaaagag     120

agtcccagcc cccaaggctc caaatggaca gggcaccatg ttgacccctt cgtcggcctc     180

cagttcgccg actgtaacat gtttcatcca gttcaggacg tttatttatt tccaacttgc     240

atacaaaaca tactg                                                      255


<210>  170
<211>  255
<212>  DNA
<213>  Homo Sapiens

<400>  170
gaatcgtcga cccacgcgtc cgcccacgcg atccgaagag atcgagctcc ctggctgccg      60

gctcgccttc tgcgtggagt tctcgcggtc tgggtttcgc tgtctgctct tggcccgggg     120

tcattttgtc ggcgtcgggt gccctctctt gcccagctgg ggcacagcga ggcggcccct     180

tctcccgacg acgttcgatg gagtagggtc ccagaccgtt gtcccgaaga gcgagatcga     240

gcttggcccc ctccc                                                      255


<210>  171
<211>  255
<212>  DNA
<213>  Homo Sapiens

<400>  171
gaatcgtcga cccacgcgtc cgcctccttc catctcatct ctatctctgg gctggtttgt      60

gagctgaaag agttcacaca tacctggggt gggcctctgt ctcctcgaca gagtgggact     120

gaggagcgag gcctgaagca ttactggttc acatcctggc ccgaccagaa gaccccagac    180

cgggcccccc cactcctgca cctggtgcgg gaggtggagg aggcagccca gcaggagggg    240

ccccactgtg ccccc                                                     255

<210>  172
<211>  255
<212>  DNA
<213>  Homo Sapiens

<220>
<221>  misc_feature
<222>  (25)..(25)
<223>  n is a, c, g, or t

<220>
<221>  misc_feature
<222>  (27)..(27)
<223>  n is a, c, g, or t

<220>
<221>  misc_feature
<222>  (32)..(32)
<223>  n is a, c, g, or t

<220>
<221>  misc_feature
<222>  (61)..(61)
<223>  n is a, c, g, or t

<220>
<221>  misc_feature
<222>  (83)..(83)
<223>  n is a, c, g, or t

<220>
<221>  misc_feature
<222>  (88)..(88)
<223>  n is a, c, g, or t

<220>
<221>  misc_feature
<222>  (96)..(96)
<223>  n is a, c, g, or t

<220>
<221>  misc_feature
<222>  (114)..(114)
<223>  n is a, c, g, or t

<220>
<221>  misc_feature
<222>  (116)..(116)
<223>  n is a, c, g, or t

<220>
<221>  misc_feature
<222>  (123)..(123)
<223>  n is a, c, g, or t

<220>
<221>  misc_feature
<222>  (144)..(144)
<223>  n is a, c, g, or t

<220>
<221>  misc_feature
<222>  (153)..(153)
<223>  n is a, c, g, or t

<220>
<221>  misc_feature
<222>  (164)..(164)
<223>  n is a, c, g, or t

<220>
<221>  misc_feature
<222>  (166)..(166)
<223>  n is a, c, g, or t

<220>
<221>  misc_feature
<222>  (174)..(174)
<223>  n is a, c, g, or t

<220>
<221>  misc_feature
<222>  (192)..(193)
<223>  n is a, c, g, or t

<220>
<221>  misc_feature
<222>  (214)..(214)
<223>  n is a, c, g, or t

<220>
<221>  misc_feature
<222>  (224)..(224)
<223>  n is a, c, g, or t

<220>
<221>  misc_feature
<222>  (233)..(233)
<223>  n is a, c, g, or t

<220>
<221>  misc_feature
<222>  (241)..(242)
<223>  n is a, c, g, or t

<220>
<221>  misc_feature
<222>  (247)..(247)
<223>  n is a, c, g, or t

<220>
<221>  misc_feature
<222>  (254)..(254)
<223>  n is a, c, g, or t

<400>  172
gaatcgtcga cccacgcgtc cgccnanacg tncgcccacg ctttgcgcga taagtgctcg      60

nccgcctttg tcctgcctac tgnatgcngg cgctgngtct gtggggctct ggcngnggcc 120

tgnccctggg acggacagca tgtnctcgtc ccnggaggac tggntntgtg cgcnctccat 180

gcacgagttt tnngccaagg agatcaacgg gcanatggtt taanctgaac aantaccggg 240

nnttctngtg catng 255

<210> 173
<211> 255
<212> DNA
<213> Homo Sapiens

<400> 173
gaatcgtcga cccacgcgtc cgggggcgcg gcctccggcg gaggagggaa cagctgggac 60

ctcccgggtt cccacgtgcg gctgccgggg cgtgcacagt ctgggacccg tggcggtgct 120

ggcaacacaa gcaccagctg cggggactcc aacagcatct gcccggcccc ctccacgatg 180

tccaaggccg aggaggccaa gaagctggcg ggccgcgcgg ctgtggagaa ccacgtgagg 240

aataaccaag tgctg 255

<210> 174
<211> 255
<212> DNA
<213> Homo Sapiens

<400> 174
gaatcgtcga cccacgcgtc cgggaattcc agatggtcaa ataaaaaaaa tgttcctaaa 60

cttggtgata tgaattcatc atttgaagat gtagatatat tttattcttt ctggtataat 120

tttgattctt ggagagaatt ttcttattta gatgaagaag aaaaagaaaa agcagaatgt 180

cgtgatgaga ggagatggat tgaaaagcag aacagagcaa caagagcaca aagaaaaaaa 240

gaagaaatga acaga 255